(19) Europäisches Patentamt / European Patent Office / Office européen des brevets

(11) EP 2 995 632 A1

(12) **EUROPEAN PATENT APPLICATION**
published in accordance with Art. 153(4) EPC

(43) Date of publication:
**16.03.2016 Bulletin 2016/11**

(21) Application number: **14795250.1**

(22) Date of filing: **07.05.2014**

(51) Int Cl.:
***C08G 59/24*** *(2006.01)* ***C07C 41/16*** *(2006.01)*
***C07C 43/162*** *(2006.01)* ***C07D 303/28*** *(2006.01)*
***C08G 59/68*** *(2006.01)*

(86) International application number:
**PCT/JP2014/062217**

(87) International publication number:
**WO 2014/181787 (13.11.2014 Gazette 2014/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **10.05.2013 JP 2013099967**
**10.05.2013 JP 2013100071**

(71) Applicant: **Daicel Corporation**
**Osaka-shi, Osaka 530-0011 (JP)**

(72) Inventors:
- **NAKAMURA, Ryota**
**Ohtake-shi**
**Hiroshima 739-0695 (JP)**
- **TANIDA, Daisuke**
**Himeji-shi**
**Hyogo 671-1283 (JP)**
- **KITAO, Kyuhei**
**Himeji-shi**
**Hyogo 671-1283 (JP)**
- **KOBATAKE, Takanori**
**Ohtake-shi**
**Hiroshima 739-0695 (JP)**

(74) Representative: **Grünecker Patent- und Rechtsanwälte**
**PartG mbB**
**Leopoldstraße 4**
**80802 München (DE)**

# (54) CURABLE EPOXY RESIN COMPOSITION AND CURED PRODUCT THEREOF, DIOLEFIN COMPOUND AND PRODUCTION METHOD THEREFOR, AND PRODUCTION METHOD FOR DIEPOXY COMPOUND

(57) An object of the present invention is to provide a curable epoxy resin composition, which is cured to provide a cured product having a high glass-transition temperature and particularly having excellent balance between heat resistance and transparency.

The present invention relates to a curable epoxy resin composition comprising an alicyclic epoxy compound (A) represented by the following formula (1) and a curing agent (B), or a curable epoxy resin composition comprising an alicyclic epoxy compound (A) represented by the following formula (1) and a curing catalyst (C). [Formula 1]

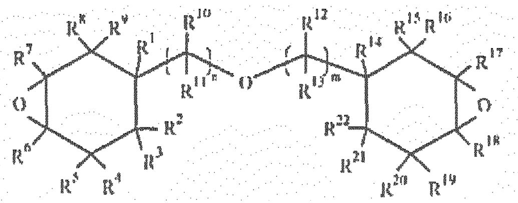

(1)

wherein $R^1$ to $R^{22}$, which may be the same or different, each represent a hydrogen atom, a methyl group or an ethyl group; and m and n, which may be the same or different, each represent an integer of 1 to 4.

EP 2 995 632 A1

## Description

### Technical Field

The present invention relates to a curable epoxy resin composition and a cured product thereof. The present invention further relates to a novel diolefin compound and a method for producing the diolefin compound, and a method for producing a diepoxy compound using the diolefin compound. The present application claims the priority rights of Japanese Patent Application No. 2013-099967, filed May 10, 2013 to the Japan Patent Office and Japanese Patent Application No. 2013-100071, filed May 10, 2013 to the Japan Patent Office, the contents of which are incorporated herein.

### Background Art

It is known that a curable epoxy resin composition comprising an epoxy compound as an essential component is cured to provide a cured product (resin cured product) excellent in e.g., electrical characteristics, moisture resistance and heat resistance. Such a curable epoxy resin composition is applied to various uses including a coating agent, a hard coating agent, an ink, an adhesive, a sealant, a sealing agent, a resist, a composite material, a transparent substrate, a transparent film or sheet, an optical material (e.g., an optical lens), an insulating material, a stereolithographic material and an electronic material (e.g., an electronic paper, a touch panel, a solar cell substrate, an optical waveguide, a light guide plate, a holographic memory).

As the curable epoxy resin composition, for example, a composition comprising an alicyclic epoxy compound such as 3,4-epoxycyclohexylmethyl(3,4-epoxy)cyclohexane carboxylate, as an essential component, is known (e.g., Patent Literatures 1 to 3). Such a curable epoxy resin composition, since it comprises an alicyclic epoxy compound, is known to provide a cured product having excellent heat resistance.

### Citation List

### Patent Literature

Patent Literature 1: Japanese Patent Laid-Open No. 63-264625
Patent Literature 2: Japanese Patent Laid-Open No. 63-012623
Patent Literature 3: Japanese Patent Laid-Open No. 59-011317

### Summary of Invention

### Technical Problem

Recently, a curable epoxy resin composition has been increasingly used in various fields. Along with this, the levels of properties (such as heat resistance and transparency) required for a cured product have been increased. Because of this, cured products obtained from the curable epoxy resin compositions described in Patent Literatures 1 to 3 mentioned above are not sufficient in heat resistance and transparency depending upon the use. In addition to this problem, the curable epoxy resin compositions have a problem in that the cure rate is not sufficiently high. From this, it is difficult to expect a further improvement in productivity.

Accordingly, an object of the present invention is to provide a curable epoxy resin composition, which is cured to provide a cured product having a high glass-transition temperature and particularly having excellent balance between heat resistance and transparency, and provide a cured product thereof.

Another object of the present invention is to provide a curable epoxy resin composition having a high cure rate, which is cured to provide a cured product having a high glass-transition temperature and particularly having excellent balance between heat resistance and transparency, and provide a cured product thereof.

Another object of the present invention is to provide a novel diolefin compound useful as a raw material for a diepoxy compound, which is an essential component for a curable epoxy resin composition as mentioned above, and provide a method for producing such a diolefin compound.

Another object of the present invention is to provide a method for producing a diepoxy compound as mentioned above.

Solution to Problem

The present inventors have conducted intensive studies with a view to solving the aforementioned problems. As a result, they found that even in a diepoxy compound having two alicyclic epoxy groups (which are each a cyclic group formed by binding each of two carbon atoms of an alicyclic ring to a single oxygen atom) in one molecule, the two alicyclic epoxy groups cannot be always present in the positions suitable for a cross-linking reaction; and that if an alicyclic epoxy group that cannot be involved in the cross-linking reaction is present, a cured product having sufficient heat resistance cannot be obtained in some cases. They also found that, in a diepoxy compound having a structure where two alicyclic epoxy groups are flexibly bound via a specific linking group, alicyclic epoxy groups can be disposed in positions suitable for a cross-linking reaction, with the result that the number of alicyclic epoxy groups not involved in the cross-linking reaction can be significantly reduced; and that a curable epoxy resin composition comprising such a specific diepoxy compound (alicyclic epoxy compound) and a curing agent as essential components is cured to provide a cured product having a high glass-transition temperature and particularly having excellent balance between heat resistance and transparency. They further found that a curable epoxy resin composition comprising the diepoxy compound and a curing catalyst as essential components has a high cure rate, and that the composition is cured to provide a cured product having a high glass-transition temperature and particularly having excellent balance between heat resistance and transparency. They moreover found that the above diepoxy compound can be obtained by oxidizing a diolefin compound having a structure where two alicyclic olefins are flexibly bound via a specific linking group. The present invention has been achieved based on these findings.

More specifically, the present invention provides a curable epoxy resin composition comprising an alicyclic epoxy compound (A) represented by the following formula (1) :

[Formula 1]

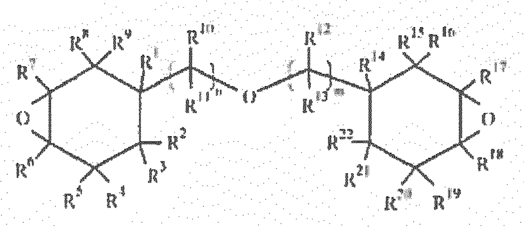

(1)

wherein $R^1$ to $R^{22}$, which may be the same or different, each represent a hydrogen atom, a methyl group or an ethyl group; and m and n, which may be the same or different, each represent an integer of 1 to 4,
and a curing agent (B).

The present invention provides a curable epoxy resin composition comprising an alicyclic epoxy compound (A) represented by the following formula (1):

[Formula 2]

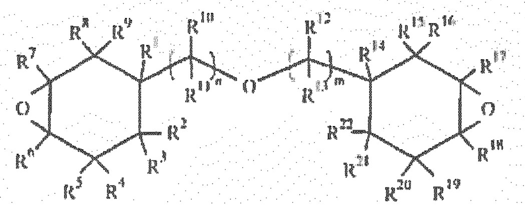

(1)

wherein $R^1$ to $R^{22}$, which may be the same or different, each represent a hydrogen atom, a methyl group or an ethyl group; and m and n, which may be the same or different, each represent an integer of 1 to 4,
and a curing catalyst (C).

The present invention provides the curable epoxy resin composition mentioned above, further comprising a curing accelerator (D).

The present invention provides a cured product obtained by curing the curable epoxy resin composition mentioned above.

The present invention provides a diolefin compound represented by the following formula (2):

[Formula 3]

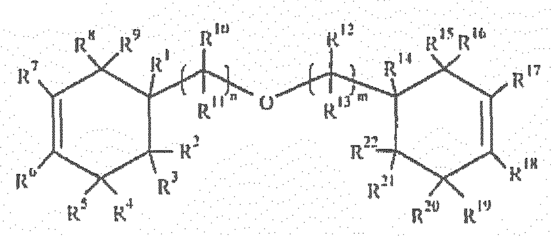

(2)

wherein $R^1$ to $R^{22}$, which may be the same or different, each represent a hydrogen atom, a methyl group or an ethyl group; and m and n, which may be the same or different, each represent an integer of 1 to 4.

The present invention provides a method for producing a diolefin compound represented by the following formula (2): where $R^1$ to $R^{22}$, m and n are the same as defined above by reacting a compound represented by the following formula (3):

[Formula 4]

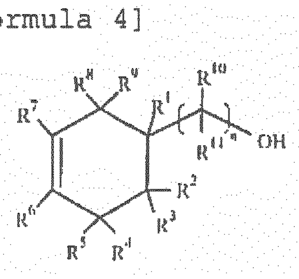

(3)

wherein $R^1$ to $R^{11}$, which may be the same or different, each represent a hydrogen atom, a methyl group or an ethyl group; and n represents an integer of 1 to 4, and a compound represented by the following formula (4):

[Formula 5]

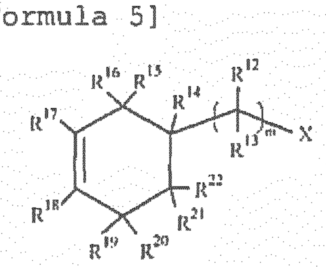

(4)

wherein $R^{12}$ to $R^{22}$, which may be the same or different, each represent a hydrogen atom, a methyl group or an ethyl group; X represents a halogen atom, a benzenesulfonyloxy group, a p-toluenesulfonyloxy group, a methanesulfonyloxy group or a trifluoromethanesulfonyloxy group; and m represents an integer of 1 to 4.

The present invention provides the method for producing a diolefin compound wherein the reaction is carried out in the presence of a basic compound.

The present invention provides a method for producing a diepoxy compound represented by the following formula (1):

[Formula 8]

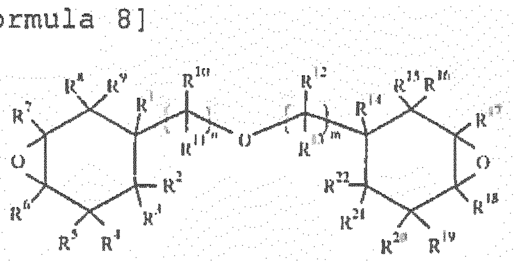

(1)

wherein $R^1$ to $R^{22}$, m and n are the same as defined above, by reacting a diolefin compound represented by the following formula (2):

[Formula 7]

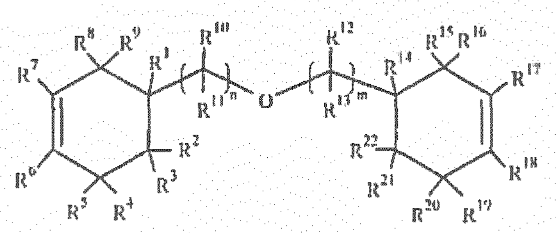

(2)

wherein $R^1$ to $R^{22}$, which may be the same or different, each represent a hydrogen atom, a methyl group or an ethyl group; and m and n, which may be the same or different, each represent an integer of 1 to 4,
and a peracid.

More specifically, the present invention relates to the followings.

[1] A curable epoxy resin composition comprising an alicyclic epoxy compound (A) represented by the above formula (1), wherein $R^1$ to $R^{22}$, which may be the same or different, each represent a hydrogen atom, a methyl group or an ethyl group; and m and n, which may be the same or different, each represent an integer of 1 to 4, and a curing agent (B).

[2] A curable epoxy resin composition comprising an alicyclic epoxy compound (A) represented by the above formula (1), wherein $R^1$ to $R^{22}$, which may be the same or different, each represent a hydrogen atom, a methyl group or an ethyl group; and m and n, which may be the same or different, each represent an integer of 1 to 4, and a curing catalyst (C).

[3] The curable epoxy resin composition according to [1] or [2], wherein the content (blending quantity) of the alicyclic epoxy compound (A) relative to the curable epoxy resin composition (100 wt%) is 0.1 wt% or more and less than 100 wt%.

[4] The curable epoxy resin composition according to [1] or [3], wherein the content (blending quantity) of the alicyclic epoxy compound (A) relative to the curable epoxy resin composition (100 wt%) is 5 to 90 wt%.

[5] The curable epoxy resin composition according to [2] or [3], wherein the content (blending quantity) of the alicyclic epoxy compound (A) relative to the curable epoxy resin composition (100 wt%) is 20 to 99.9 wt%.

[6] The curable epoxy resin composition according to any one of [1] to [5], wherein the content (blending quantity) of the alicyclic epoxy compound (A) relative to the total amount (100 wt%) of cationic curable compounds comprised in the curable epoxy resin composition is 1 to 100 wt%.

[7] The curable epoxy resin composition according to any one of [1] to [6], wherein $R^1$ to $R^{22}$ all represent a hydrogen atom.

[8] The curable epoxy resin composition according to any one of [1] to [7], wherein m and n each represent 1.

[9] The curable epoxy resin composition according to any one of [1], [3], [4] and [6] to [8], wherein the content (blending quantity) of the curing agent (B) relative to the total amount (100 parts by weight) of cationic curable compounds comprised in the curable epoxy resin composition is 50 to 200 parts by weight.

[10] The curable epoxy resin composition according to any one of [1], [3], [4] and [6] to [9], wherein the curing agent (B) is an acid anhydride.

[11] The curable epoxy resin composition according to [10], wherein the acid anhydride is an anhydride of a saturated monocyclic hydrocarbon dicarboxylic acid (including a saturated monocyclic hydrocarbon dicarboxylic acid having a substituent such as an alkyl group attached to the ring).

[12] The curable epoxy resin composition according to any one of [2], [3] and [5] to [8], wherein the content (blending quantity) of the curing catalyst (C) relative to the total amount (100 parts by weight) of cationic curable compounds comprised in the curable epoxy resin composition is 0.01 to 15 parts by weight.

[13] The curable epoxy resin composition according to any one of [1] to [12], further comprising a curing accelerator (D).

[14] The curable epoxy resin composition according to [13], wherein the content (blending quantity) of the curing accelerator (D) relative to the total amount (100 parts by weight) of cationic curable compounds comprised in the curable epoxy resin composition is 0.01 to 5 parts by weight.

[15] A cured product obtained by curing any one of the curable epoxy resin compositions [1] to [14].

[16] A diolefin compound represented by the above formula (2) wherein $R^1$ to $R^{22}$, which may be the same or different, each represent a hydrogen atom, a methyl group or an ethyl group; and m and n, which may be the same

or different, each represent an integer of 1 to 4.

[17] The diolefin compound according to [16], wherein $R^1$ to $R^{22}$ all represent a hydrogen atom.

[18] The diolefin compound according to [16] or [17], wherein m and n each represent 1.

[19] A method for producing a diolefin compound represented by the above formula (2) wherein $R^1$ to $R^{22}$, m and n are the same as defined above, by reacting a compound represented by the above formula (3), wherein $R^1$ to $R^{11}$, which may be the same or different, each represent a hydrogen atom, a methyl group or an ethyl group; and n represents an integer of 1 to 4, and a compound represented by the above formula (4), wherein $R^{12}$ to $R^{22}$, which may be the same or different, each represent a hydrogen atom, a methyl group or an ethyl group; X represents a halogen atom, a benzenesulfonyloxy group, a p-toluenesulfonyloxy group, a methanesulfonyloxy group or a trifluoromethanesulfonyloxy group; and m represents an integer of 1 to 4.

[20] The method for producing a diolefin compound according to [19], wherein the reaction is carried out in the presence of a basic compound.

[21] The method for producing a diolefin compound according to [20], wherein the amount of basic compound used relative to one mole of a compound represented by the formula (3) is 0.5 to 10 moles.

[22] The method for producing a diolefin compound according to any one of [19] to [21], wherein the temperature of the reaction is 0 to 150°C.

[23] The method for producing a diolefin compound according to any one of [19] to [22], wherein the time for the reaction is 0.5 to 5 hours.

[24] The method for producing a diolefin compound according to any one of [19] to [23], wherein a compound represented by the formula (3) is reacted with a halogenating agent or a compound represented by the following formula (5) to produce a compound represented by the formula (4), and thereafter, the above reaction (reaction between a compound represented by the formula (3) and a compound represented by the formula (4)) is carried out.

[25] The method for producing a diolefin compound according to [24], wherein the reaction (reaction for obtaining a compound represented by the formula (4)) is carried out in the presence of a basic compound.

[26] The method for producing a diolefin compound according to [25], wherein the amount of basic compound used in the reaction for obtaining a compound represented by the formula (4) relative to one mole of a compound represented by the formula (3) is 0.5 to 10 moles.

[27] The method for producing a diolefin compound according to any one of [19] to [26], wherein $R^1$ to $R^{22}$ all represent a hydrogen atom.

[28] The method for producing a diolefin compound according to any one of [19] to [27], wherein m and n each represent 1.

[29] A method for producing a diepoxy compound represented by the above formula (1), wherein $R^1$ to $R^{22}$, m and n are the same as defined above, by reacting a diolefin compound represented by the above formula (2), wherein $R^1$ to $R^{22}$, which may be the same or different, each represent a hydrogen atom, a methyl group or an ethyl group; and m and n, which may be the same or different, each represent an integer of 1 to 4, and a peracid.

[30] The method for producing a diepoxy compound according to [29], wherein the amount of peracid used relative to one mole of a diolefin compound represented by the formula (2) is 1 to 10 moles.

[31] The method for producing a diepoxy compound according to [29] or [30], wherein the temperature of the reaction is 0 to 80°C.

[32] The method for producing a diepoxy compound according to any one of [29] to [31], wherein the time for the reaction is 2 to 10 hours.

[33] The method for producing a diepoxy compound according to any one of [29] to [32], wherein $R^1$ to $R^{22}$ all represent a hydrogen atom.

[34] The method for producing a diepoxy compound according to any one of [29] to [33], wherein m and n each represent 1.

Advantageous Effects of Invention

The curable epoxy resin composition of the present invention, since it has the aforementioned constitution, is cured to provide a cured product having a high glass-transition temperature and particularly having excellent balance between heat resistance and transparency. Of them, particularly in the case where the curable epoxy resin composition of the present invention comprises a curing catalyst as an essential component, since the composition has a high cure rate, a cured product can be provided with high productivity.

A diolefin compound of the present invention (a diolefin compound represented by the formula (2)) has a structure where two alicyclic olefins are flexibly bound via a specific linking group. Thus, in a diepoxy compound obtained by oxidizing the diolefin compound, alicyclic epoxy groups can be appropriately disposed in positions suitable for a cross-linking reaction, with the result that the ratio of alicyclic epoxy groups not involved in the cross-linking reaction can be significantly reduced. Because of this, a densely cross-linked structure can be obtained by polymerization and a cured

product having excellent heat resistance and successfully maintaining excellent mechanical characteristics even if exposed to a high-temperature environment can be obtained. In short, the diolefin compound of the present invention is extremely useful as a raw material for a diepoxy compound providing a cured product having excellent heat resistance.

When a diepoxy compound obtained by oxidizing a diolefin compound of the present invention is polymerized, a densely cross-linked structure can be formed and a cured product having excellent heat resistance can be obtained. Because of this, the diepoxy compound can be preferably applied to various uses including a coating agent, a hard coating agent, an ink, an adhesive, a sealant, a sealing agent, a resist, a composite material, a transparent substrate, a transparent film or sheet, an optical material (e.g., optical lens), an insulating material, a stereolithographic material and an electronic material (e.g., an electronic paper, a touch panel, a solar cell substrate, an optical waveguide, a light guide plate, a holographic memory).

Brief Description of Drawing

[Figure 1] Figure 1 shows measurement results (DSC curve) of photochemical reaction heat generated by curable epoxy resin compositions obtained in Example 8 and Comparative Example 7. The solid line shows the DSC curve of the curable epoxy resin composition obtained in Example 8; whereas the dashed line shows the DSC curve of the curable epoxy resin composition obtained in Comparative Example 7.

Description of Embodiments

<Curable epoxy resin composition>

The curable epoxy resin composition of the present invention is a curable epoxy resin composition comprising an alicyclic epoxy compound (A) represented by the following formula (1) (sometimes, simply referred to as an "alicyclic epoxy compound (A)") and a curing agent (B) as essential components or a curable epoxy resin composition comprising an alicyclic epoxy compound (A) and a curing catalyst (C) as essential components. The curable epoxy resin composition of the present invention may further comprise components other than the aforementioned essential components (components (A) to (C)), if necessary.

[Formula 9]

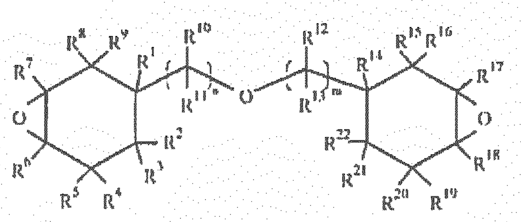

(1)

[Alicyclic epoxy compound (A)]

In the curable epoxy resin composition of the present invention, the alicyclic epoxy compound (A) is a compound represented by the above formula (1) [a diepoxy compound having two epoxy groups (alicyclic epoxy groups) in a molecule]. In the formula (1), $R^1$ to $R^{22}$, which may be the same or different, each represent a hydrogen atom, a methyl group or an ethyl group. In particular, $R^1$ to $R^{22}$ each preferably represent a hydrogen atom and particularly preferably all of $R^1$ to $R^{22}$ represent a hydrogen atom. In the formula (1), m and n, which may be the same or different, each represent an integer of 1 to 4, and in particular, m and n each represent preferably 1. Note that in the case where m and/or n represents an integer of 2 or more, two or more of $R^{10}$ to $R^{13}$ each may be the same or different.

An alicyclic diepoxy compound (A) is produced, for example, by a method of reacting a diolefin compound represented by the following formula (2) and a peracid; however, the method for producing an alicyclic diepoxy compound (A) is not particularly limited.

[Formula 10]

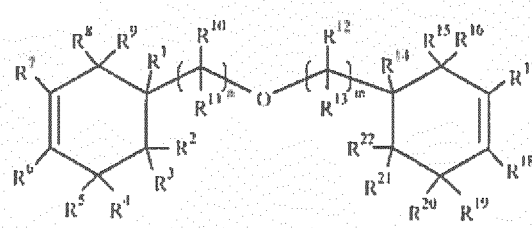

(2)

wherein $R^1$ to $R^{22}$, m and n are the same as defined above.

As the peracid, a known or conventional peracid such as performic acid, peracetic acid, perbenzoic acid, metachloroperbenzoic acid and trifluoroperacetic acid, can be used. These may be used alone or in combination of two or more.

The amount of peracid used, although it is not particularly limited, is preferably for example about 1 to 10 moles relative to one mole of a diolefin compound represented by the formula (2), and more preferably 2 to 4 moles.

The aforementioned reaction may be carried out in the presence of a solvent. Any solvent may be used as long as it does not inhibit a reaction from proceeding. Examples of the solvent include, but are not particularly limited to, an aromatic compound such as toluene and benzene; an aliphatic hydrocarbon such as hexane and cyclohexane; and an ester such as ethyl acetate. These may be used alone or as a mixture of two or more.

The atmosphere of the aforementioned reaction is not particularly limited as long as it does not inhibit the reaction. For example, a nitrogen atmosphere or an argon atmosphere may be used. The temperature of the reaction, although it is not particularly limited, is, for example, about 0 to 80°C and preferably 20 to 50°C. The time for the reaction, although it is not particularly limited, is, for example, about 2 to 10 hours.

After completion of the reaction, a reaction product (alicyclic epoxy compound (A)) can be separated and purified, by a separation and purification means such as filtration, concentration, distillation, extraction, crystallization, recrystallization, adsorption, and column chromatography, or a combination of these.

An alicyclic epoxy compound (A) (diepoxy compound) has a structure where two alicyclic epoxy groups are flexibly bound via a linking group $[-(CR^{10}R^{11})_n-O-(CR^{12}R^{13})_m-]$ (m and n, which may be the same or different, each represent an integer of 1 to 4). Thus, in polymerizing, the alicyclic epoxy groups can be appropriately disposed in positions suitable for a cross-linking reaction. Accordingly, the ratio of the alicyclic epoxy group not involved in the cross-linking reaction can be significantly reduced. Because of this, a densely cross-linked structure can be formed by a polymerization reaction using e.g., a curing agent (B) and a curing catalyst (C) and a cured product having excellent heat resistance and successfully maintaining excellent mechanical characteristics even if exposed to a high-temperature environment, can be provided. Accordingly, an alicyclic epoxy compound (A) can be preferably applied to various uses including a coating agent, a hard coating agent, an ink, an adhesive, a sealant, a sealing agent, a resist, a composite material, a transparent substrate, a transparent film or sheet, an optical material (e.g., optical lens), an insulating material, a stereolithographic material and an electronic material (e.g., an electronic paper, a touch panel, a solar cell substrate, an optical waveguide, a light guide plate, a holographic memory).

Note that a diolefin compound represented by the formula (2) can be produced by reacting, for example, a compound represented by the following formula (3):

[Formula 11]

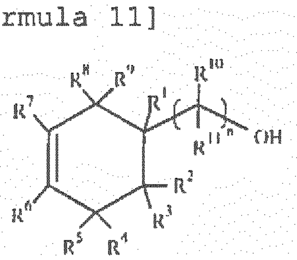

(3)

wherein $R^1$ to $R^{11}$, which may be the same or different, each represent a hydrogen atom, a methyl group or an ethyl group; and n represents an integer of 1 to 4, as defined above,
and a compound represented by the following formula (4):

[Formula 12]

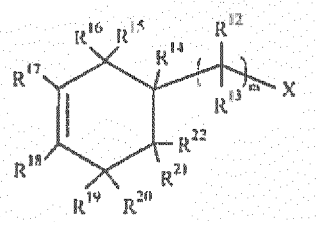

(4)

wherein $R^{12}$ to $R^{22}$, which may be the same or different, each represent a hydrogen atom, a methyl group or an ethyl group, as defined above; X represents a halogen atom, a benzenesulfonyloxy group, a p-toluenesulfonyloxy group, a methanesulfonyloxy group or a trifluoromethanesulfonyloxy group; and m represents an integer of 1 to 4.

As a compound represented by the formula (3), a commercially available compound can be used.

A compound represented by the formula (4) can be produced, for example, by reacting a compound represented by the formula (3) and a halogenating agent or a compound represented by the following formula (5):

$$X^1\text{-}Y \quad (5)$$

wherein $X^1$ represents a benzenesulfonyloxy group, a p-toluenesulfonyloxy group, a methanesulfonyloxy group or a trifluoromethanesulfonyloxy group; and Y represents a halogen atom.

Examples of the above halogenating agent include a molecular halogen such as chlorine and bromine; an N-haloamide such as an N-chloroamide and an N-bromoamide; a hypohalous acid such as hypochlorous acid and hypobromous acid; a hypohalous acid ester such as t-butyl hypochlorite and t-butyl hypobromite; an N-haloimide such as N-chlorosuccinimide and N-bromosuccinimide; an anhydrous metal halide such as aluminum chloride, aluminum bromide, ferric chloride, cupric chloride and antimony chloride; BrCl, ICl and $ClO_3F$.

Examples of a compound represented by the above formula (5) include benzenesulfonyl chloride, p-toluenesulfonyl chloride, methanesulfonyl chloride, trifluoromethanesulfonyl chloride, benzenesulfonyl bromide, p-toluenesulfonyl bromide, methanesulfonyl bromide, trifluoromethanesulfonyl bromide, benzenesulfonyl iodide, p-toluenesulfonyl iodide, methanesulfonyl iodide and trifluoromethanesulfonyl iodide.

The amount of halogenating agent or compound represented by the formula (5) used, although it is not particularly limited, is preferably, for example, about 0.5 to 5 moles relative to one mole of a compound represented by the formula (3).

The reaction for obtaining a diolefin compound represented by the above formula (2) and the reaction for obtaining a compound represented by the formula (4) are preferably carried out in the presence of a base (basic compound), because a desired product can be obtained with good selectivity. In contrast, if the above reaction is carried out in acidic conditions, an intramolecular addition reaction or an intermolecular addition reaction of a compound represented by the formula (3) takes place, with the result that selectivity can be sometimes reduced. As the base, either an inorganic base or an organic base may be used. These may be used alone or in combination of two or more.

Examples of the inorganic base include an alkali metal hydroxide such as lithium hydroxide, sodium hydroxide, potassium hydroxide and cesium hydroxide; an alkali metal carbonate such as lithium carbonate, sodium carbonate, potassium carbonate and cesium carbonate; an alkali metal hydrogen carbonate such as sodium hydrogen carbonate, potassium hydrogen carbonate and cesium hydrogen carbonate; an alkaline earth metal hydroxide such as magnesium hydroxide, calcium hydroxide and barium hydroxide; an alkaline earth metal carbonate such as magnesium carbonate, calcium carbonate and barium carbonate; and cerium carbonate.

Examples of the organic base include an alkali metal alkoxide such as sodium methoxide, sodium ethoxide and potassium t-butoxide; an alkali metal salt of an organic acid such as sodium acetate; and a nitrogen-containing heterocyclic compound such as triethylamine, piperidine, N-methylpiperidine and pyridine.

The amount of base used, although it is not particularly limited, is preferably, for example, about 0.5 to 10 moles relative to one mole of a compound represented by the formula (3).

The reaction is usually carried out in the presence of a solvent inactive to the reaction. Examples of the solvent include an aromatic compound such as toluene and benzene; an aliphatic hydrocarbon such as hexane and cyclohexane; and an ester such as ethyl acetate. These may be used alone or as a mixture of two or more.

The atmosphere of the reaction is not particularly limited as long as it does not inhibit the reaction. For example, either a nitrogen atmosphere or an argon atmosphere may be used. The temperature of the reaction, although it is not

particularly limited, is, for example, about 0 to 150°C. The time for the reaction, although it is not particularly limited, is, for example, about 0.5 to 5 hours. The reaction may be carried out, for example, in any one of the batch, semi-batch and continuous systems.

After completion of the reaction, a reaction product (a diolefin compound represented by the formula (2)) can be separated and purified by a separation and purification means such as filtration, concentration, distillation, extraction, crystallization, recrystallization, adsorption and column chromatography, or a combination of these.

Note that as the alicyclic epoxy compound (A), a commercially available compound can be used.

In the curable epoxy resin composition of the present invention, compounds can be used alone or in combination of two or more, as the alicyclic epoxy compound (A).

In the curable epoxy resin composition of the present invention, the content (blending quantity) of the alicyclic epoxy compound (A), although it is not particularly limited, is preferably 0.1 wt% or more (e.g., 0.1 wt% or more, less than 100 wt%) relative to the curable epoxy resin composition (100 wt%), more preferably 1 wt% or more, and further preferably 10 wt% or more. If the content of the alicyclic epoxy compound (A) is outside the above range, the cure rate of the curable epoxy resin composition as well as the heat resistance and transparency of a cured product may be insufficient.

In the curable epoxy resin composition of the present invention, the content (blending quantity) of the alicyclic epoxy compound (A), although it is not particularly limited, is preferably, 5 to 90 wt% relative to the curable epoxy resin composition (100 wt%), if the curable epoxy resin composition of the present invention comprises a curing agent (B) as an essential component, and more preferably 15 to 80 wt%. In contrast, if the curable epoxy resin composition of the present invention comprises a curing catalyst (C) as an essential component, the content (blending quantity) of the alicyclic epoxy compound (A) relative to the curable epoxy resin composition (100 wt%) is preferably 20 to 99.9 wt% and more preferably 30 to 99.9 wt%.

The content (blending quantity) of the alicyclic epoxy compound (A) relative to the total amount of cationic curable compound (100 wt%; the total amount of alicyclic epoxy compound (A) and the other cationic curable compound (later described)) comprised in the curable epoxy resin composition, although it is not particularly limited, is preferably 1 wt% or more (e.g., 1 to 100 wt%), more preferably 10 wt% or more, further preferably 20 wt% or more, and particularly preferably 50 wt% or more. If the content of an alicyclic epoxy compound (A) is less than 1 wt%, the cure rate of the curable epoxy resin composition as well as the heat resistance and transparency of a cured product may be insufficient.

[Curing agent (B)]

In the curable epoxy resin composition of the present invention, the curing agent (B) is a compound, which plays a role of curing a curable epoxy resin composition by reacting with e.g., a cationic curable compound such as an alicyclic epoxy compound (A). As the curing agent (B), a curing agent known or conventionally used as a curing agent for an epoxy resin can be used. Examples thereof include, but are not particularly limited to, an acid anhydride (acid anhydride curing agent), an amine (amine curing agent), a polyamide resin, an imidazole (imidazole curing agent), a polymercaptan (polymercaptan curing agent), a phenol (phenol curing agent), a polycarboxylic acid, a dicyandiamide and an organic acid hydrazide.

As the acid anhydride (acid anhydride curing agent) serving as the curing agent (B), a known or conventional acid anhydride curing agent can be used. Examples thereof include, but are not particularly limited to, a methyl tetrahydrophthalic anhydride (e.g., 4-methyl-tetrahydrophthalic anhydride, 3-methyl-tetrahydrophthalic anhydride), a methylhexahydrophthalic anhydride (e.g., 4-methylhexahydrophthalic anhydride, 3-methylhexahydrophthalic anhydride), dodecenyl succinic anhydride, methyl endomethylene tetrahydrophthalic anhydride, phthalic anhydride, maleic anhydride, tetrahydrophthalic anhydride, hexahydrophthalic anhydride, methyl cyclohexene dicarboxylic acid anhydride, pyromellitic anhydride, trimellitic anhydride, benzophenonetetracarboxylic anhydrides, nadic acid anhydride, methyl nadic acid anhydride, hydrogenated methyl nadic acid anhydride, 4-(4-methyl-3-pentenyl) tetrahydrophthalic anhydride, succinic anhydride, adipic anhydride, sebacic anhydride, dodecanedioic acid anhydride, methyl cyclohexene tetracarboxylic anhydride, a vinyl ether-maleic anhydride copolymer and an alkyl styrene-maleic anhydride copolymer. Of them, in view of handling, an acid anhydride present in a liquid state at 25°C [e.g., methyl tetrahydrophthalic anhydride, methylhexahydrophthalic anhydride, dodecenyl succinic anhydride, methyl endomethylene tetrahydrophthalic anhydride] is preferable. Note that, as to an acid anhydride present in a solid state at 25°C, if it is dissolved, for example, in an acid anhydride present in a liquid state at 25°C to prepare a liquid mixture, handling as the curing agent (B) in the curable epoxy resin composition of the present invention tends to be improved. As the acid anhydride curing agent, an anhydride of a saturated monocyclic hydrocarbon dicarboxylic acid (including a compound having a ring to which a substituent such as an alkyl group is bound) is preferable in view of the heat resistance and transparency of a cured product.

As the amine (amine curing agent) serving as the curing agent (B), a known or conventional amine curing agent can be used. Examples thereof include, but not particularly limited to, an aliphatic polyamine such as ethylenediamine, diethylenetriamine, triethylenetetramine, tetraethylene pentamine, dipropylenediamine, diethylaminopropylamine and

polypropylenetriamine; a alicyclic polyamine such as menthenediamine, isophoronediamine, bis(4-amino-3-methyldicyclohexyl)methane, diamino dicyclohexyl methane, bis(aminomethyl)cyclohexane, N-aminoethylpiperazine and 3,9-bis(3-aminopropyl)-3,4,8,10-tetraoxaspiro(5,5)undecane; a mononuclear polyamine such as m-phenylenediamine, p-phenylenediamine, tolylene-2,4-diamine, tolylene-2,6-diamine, mesitylene-2,4-diamine, 3,5-diethyl-tolylene-2,4-diamine and 3,5-diethyl-tolylene-2,6-diamine; and an aromatic polyamine such as biphenylene diamine, 4,4-diaminodiphenylmethane, 2,5-naphthylenediamine and 2,6-naphthylenediamine.

As the phenol (phenolic curing agent) serving as the curing agent (B), a known or conventional phenolic curing agent can be used. Examples thereof include, but are not particularly limited to, a Novolak phenol resin, a Novolak cresol resin, an aralkyl resin such as para-xylylene modified phenol resin, para-xylylene/meta-xylylene modified phenol resin, a terpene modified phenol resin, a dicyclopentadiene modified phenol resin and a triphenol propane.

Examples of the polyamide resin serving as the curing agent (B) include a polyamide resin having one or both of a primary amino group and a secondary amino group within a molecule.

As the imidazole (imidazole curing agent) serving as the curing agent (B), a known or conventional imidazole curing agent can be used. Examples thereof include, but are not particularly limited to, 2-methylimidazole, 2-ethyl-4-methylimidazole, 2-undecylimidazole, 2-heptadecylimidazole, 2-phenylimidazole, 1-benzyl-2-methylimidazole, 2-cyanoethyl-2-methylimidazole, 1-cyanoethyl-2-ethyl-4-methylimidazole, 1-cyanoethyl-2-undecylimidazole, 1-cyanoethyl-2-undecylimidazalium trimellitate, 1-cyanoethyl-2-phenylimidazolium trimellitate, 2-methylimidazolium isocyanurate, 2-phenylimidazolium isocyanurate, 2,4-diamino-6-[2-methyl-imidazolyl-(1)]-ethyl-s-triazine and 2,4-diamino-6-[2-ethyl-4-methylimidazolyl-(1)]-ethyl-s-triazine.

Examples of the polymercaptan (polymercaptan curing agent) serving as the curing agent (B) include a liquid-state polymercaptan and a polysulfide resin.

Examples of the poly-carboxylic acid serving as the curing agent (B) include adipic acid, sebacic acid, terephthalic acid, trimellitic acid and a carboxyl group-containing polyester.

Of them, as the curing agent (B), an acid anhydride (acid anhydride curing agent) is preferable in view of the heat resistance and transparency of a cured product. Note that in the curable epoxy resin composition of the present invention, compounds can be used alone or in combination of two or more, as the curing agent (B). As the curing agent (B), a commercially available compound can be used. Examples of the commercially available compound serving as an acid anhydride curing agent include "RIKACID MH-700" and "RIKACID MH-700F" (trade names, manufactured by New Japan Chemical Co., Ltd.); and "HN-5500" (trade name, manufactured by Hitachi Chemical Co., Ltd.).

In the curable epoxy resin composition of the present invention, the content (blending quantity) of the curing agent (B), although it is not particularly limited, is preferably 50 to 200 parts by weight relative to the total amount (100 parts by weight) of cationic curable compounds comprised in the curable epoxy resin composition, and more preferably 80 to 150 parts by weight. More specifically, in the case where an acid anhydride is used as the curing agent (B), the acid anhydride is preferably used in a ratio of 0.5 to 1.5 equivalents per epoxy group (one equivalent) in all compounds having an epoxy group in the curable epoxy resin composition of the present invention. If the content of the curing agent (B) is less than 50 parts by weight, curing becomes insufficient and the toughness of a cured product tends to reduce. In contrast, if the content of the curing agent (B) exceeds 200 parts by weight, a cured product is stained and the hue may sometimes deteriorate.

[Curing catalyst (C)]

In the curable epoxy resin composition of the present invention, the curing catalyst (C) is a compound having a function of curing a curable epoxy resin composition by initiating and/or promoting a curing reaction (polymerization reaction) of a cationic curable compound such as an alicyclic epoxy compound (A). Examples of the curing catalyst (C) include, but are not particularly limited to, a cationic polymerization initiator (e.g., photocationic polymerization initiator, thermal cationic polymerization initiator), which initiates polymerization by generating cationic species upon e.g., light irradiation and heat treatment, a Lewis acid-amine complex, a Bronsted acid salt and an imidazole.

Examples of the photocationic polymerization initiator serving as the curing catalyst (C) include a hexafluoroantimonate, a pentafluoro-hydroxyantimonate and a hexafluorophosphate and a hexafluoroarsenate. Specific examples thereof include a sulfonium salt (in particular, triarylsulfonium salts) such as triarylsulfonium hexafluorophosphate (e.g., p-phenylthiophenyldiphenylsulfonium hexafluorophosphate), triarylsulfonium hexafluoroantimonate; an iodonium salt such as diaryliodonium hexafluorophosphate, diaryliodonium hexafluoroantimonate, bis(dodecylphenyl)iodonium tetrakis(pentafluorophenyl) borate, and iodonium[4-(4-methylphenyl-2-methylpropyl)phenyl] hexafluorophosphate; a phosphonium salt such as tetrafluorophosphonium hexafluorophosphate; and a pyridinium salt such as N-hexylpyridinium tetrafluoroborate. As the photocationic polymerization initiator, for example, commercially available products such as "UVACURE 1590" (trade name, manufactured by Daicel-Cytec Company Ltd.);"CD-1010", "CD-1011" and "CD-1012" (trade names, manufactured by Sartomer USA); "IRGACURE 264" (trade name, manufactured by BASF); "CIT-1682" (trade name, manufactured by Nippon Soda Co., Ltd.); and "CPT-100P" (trade name, manufactured by San-Apro Ltd.)

can be preferably used.

Examples of the thermal cationic polymerization initiator serving as the curing catalyst (C) include an aryldiazonium salt, an aryliodonium salt, an arylsulfonium salt and an allen-ion complex, and commercially available products such as "PP-33", "CP-66" and "CP-77" (trade names, manufactured by ADEKA CORP.); "FC-509" (trade name, manufactured by 3M); "UVE1014" (trade name, manufactured by G. E.); "Sunaid SI-60L", "Sunaid SI-80L", "Sunaid SI-100L", "Sunaid SI-110L" and "Sunaid SI-150L" (trade names, manufactured by Sanshin Chemical Industry Co., Ltd.); and "CG-24-61" (trade name, manufactured by BASF) can be preferably used. Further, examples of the thermal cationic polymerization initiator include a compound between a chelate compound, which is obtained from a metal such as aluminum and titanium and acetoacetic acid or a diketone, and a silanol such as triphenylsilanol or a compound between a chelate compound, which is obtained from a metal such as aluminum and titanium and acetoacetic acid or a diketone, and a phenol such as bisphenol S.

As the Lewis acid-amine complex serving as the curing catalyst (C), a known or conventional Lewis acid-amine complex based curing catalyst can be used. Examples thereof include, but are not particularly limited to, $BF_3$·n-hexylamine, $BF_3$-monoethylamine, $BF_3$-benzylamine, $BF_3$·diethylamine, $BF_3$·piperidine, $BF_3$·triethylamane, $BF_3$·aniline, $BF_4$·n-hexylamine, $BF_4$·monoethylamine, $BF_4$·benzylamine, $BF_4$·diethylamine, $BF_4$·piperidine, $BF_4$·triethylamine, $BF_4$.aniline, $PF_5$·ethylamine, $PF_5$·isopropylamine, $PF_5$·butylamine, $PF_5$·laurylamine, $PF_5$·benzylamine and $AsF_5$·laurylamine.

As the Bronsted acid salts serving as the curing catalyst (C), a known or conventional Bronsted acid salt can be used. Examples thereof include, but are not particularly limited to, an aliphatic sulfonium salt, an aromatic sulfonium salt, an iodonium salt and a phosphonium salt.

As the imidazole serving as the curing catalyst (C), a known or conventional imidazole can be used. Examples thereof include, but are not particularly limited to, 2-methylimidazole, 2-ethyl-4-methylimidazole, 2-undecylimidazole, 2-heptadecylimidazole, 2-phenylimidazole, 1-benzyl-2-methylimidazole, 1-cyanoethyl-2-methylimidazole, 1-cyanoethyl-2-ethyl-4-methylimidazole, 1-cyanoethyl-2-undecylimidazole, 1-cyanoethyl-2-undecyl imidazolium trimellitate, 1-cyanoethyl-2-phenylimidazolium trimellitate, 2-methylimidazolium isocyanurate, 2-phenylimidazolium isocyanurate, 2,4-diamino-6-(2-methyl-imidazolyl-(1))-ethyl-s-triazine and 2,4-diamino-6-[2-ethyl-4-methylimidazolyl-(1)]-ethyl-s-triazine.

In the curable epoxy resin composition of the present invention, compounds can be used alone or in combination of two or more as the curing catalyst (C).

In the curable epoxy resin composition of the present invention, the content (blending quantity) of the curing catalyst (C), although it is not particularly limited, is preferably 0.01 to 15 parts by weight relative to the total amount (100 parts by weight) of cationic curable compounds comprised in the curable epoxy resin composition, more preferably 0.01 to 12 parts by weight, further preferably 0.05 to 10 parts by weight, and particularly preferably 0.05 to 8 parts by weight. If the curing catalyst (C) is used within the above range, the cure rate of the curable epoxy resin composition increases and the balance between heat resistance and transparency of a cured product tends to be improved.

[Curing accelerator (D)]

Particularly in the case where the curable epoxy resin composition of the present invention comprises the curing agent (B), it is preferable that the curable epoxy resin composition further comprise a curing accelerator (D). The curing accelerator (D) is a compound having a function of accelerating a rate of a reaction between a cationic curable compound (particularly, a compound having an epoxy group) and the curing agent (B). As the curing accelerator (D), a known or conventional curing accelerator can be used. Examples thereof include 1,8-diazabicyclo[5.4.0]undecene-7 (DBU) or a salt thereof (e.g., a phenol salt, an octylate, p-toluenesulfonate, a formate, a tetraphenylborate); 1,5-diazabicyclo[4.3.0]nonene-5 (DBN) or a salt thereof (e.g., a phenol salt, octylate, p-toluenesulfonate, formate, tetraphenylborate); a tertiary amine such as benzyldimethylamine, 2,4,6-tris(dimethylaminomethyl)phenol and N,N-dimethylcyclohexylamine; an imidazole such as 2-ethyl-4-methylimidazole and 1-cyanoethyl-2-ethyl-4-methylimidazole; a phosphate ester; a phosphine such as triphenyl phosphine and tris(dimethoxy)phosphine; a phosphonium compound such as tetraphenylphosphonium tetra(p-tolyl)borate; an organometallic salt such as zinc octylate, tin octylate and zinc stearate; and a metal chelate such as aluminum acetylacetone complex. As the curing accelerator (D), compounds can be used alone or in combination of two or more.

As the curing accelerator (D), a commercially available product such as "U-CAT SA 506", "U-CAT SA 102", "U-CAT 5003", "U-CAT 18X" (trade names) and "U-CAT 12XD" (development product) (manufactured by San-Apro Ltd.); "TPP-K" and "TPP-MK" (trade names, manufactured by HOKKO CHEMICAL INDUSTRY CO., Ltd.); and "PX-4ET" (trade name, manufactured by Nippon Chemical Industrial Co., Ltd.) can be used.

In the curable epoxy resin composition of the present invention, the content (blending quantity) of the curing accelerator (D), although it is not particularly limited, is preferably 0.01 to 5 parts by weight relative to the total amount (100 parts by weight) of cationic curable compounds comprised in the curable epoxy resin composition, more preferably 0.03 to 3 parts by weight and further preferably 0.03 to 2 parts by weight. If the content of the curing accelerator (D) is

less than 0.01 parts by weight, the curing acceleration effect can be sometimes insufficient. In contrast, if the content of the curing accelerator (D) exceeds 5 parts by weight, a cured product is stained and the hue may sometimes deteriorate.

[Other cationic curable compound]

The curable epoxy resin composition of the present invention may comprise another cationic curable compound (sometimes referred to as "the other cationic curable compound") except the alicyclic epoxy compound (A). Examples of the other cationic curable compound include an alicyclic epoxy compound except the alicyclic epoxy compound (A), an aromatic glycidyl ether epoxy compound, an aliphatic polyhydric alcohol polyglycidyl ether, an oxetane compound (oxetanyl compound) and a vinyl ether compound (compound having a vinyl ether group).

Specific examples of the alicyclic epoxy compound except the alicyclic epoxy compound (A) include (i) a compound except an alicyclic epoxy compound (A), having an epoxy group (alicyclic epoxy group) constituted of two adjacent carbon atoms of an alicyclic ring and an oxygen atom, (ii) a compound in which an epoxy group is directly bound to an alicyclic ring via a single bond and (iii) a compound having an alicyclic ring and a glycidyl group.

As the compound (i) except the alicyclic epoxy compound (A), having an epoxy group (alicyclic epoxy group) constituted of two adjacent carbon atoms of an alicyclic ring and an oxygen atom, a compound arbitrarily selected from known or conventional compounds can be used. As the alicyclic epoxy group, a cyclohexene oxide group is preferable.

As the compound (i) except the alicyclic epoxy compound (A), having an epoxy group constituted of two adjacent carbon atoms of an alicyclic ring and an oxygen atom, a compound having a cyclohexene oxide group is preferable, in view of transparency and heat resistance, and particularly, a compound (alicyclic epoxy compound) represented by the following formula (I) is preferable.

[Formula 13]

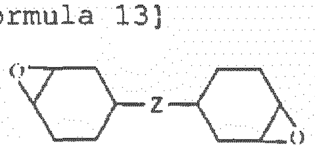

(I)

In the above formula (I), Z represents a single bond or a linking group (a divalent group having one or more atoms). Examples of the above linking group include a divalent hydrocarbon group, an alkenylene group whose carbon-carbon double bonds are wholly or partially epoxidized, a carbonyl group, an ether bond, an ester bond, a carbonate group, an amide group and a group formed by linking a plurality of these. However, a group represented by the following formula (6) is not included in the above linking group.

[Formula 14]

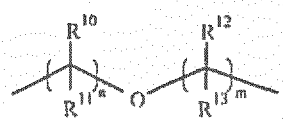

(6)

wherein $R^{10}$ to $R^{13}$, m and n are the same as defined in formula (1).

Examples of a compound represented by the above formula (I) wherein Z represents a single bond include 3,4,3',4'-diepoxybicyclohexane.

Examples of the above divalent hydrocarbon group include a linear or branched alkylene group having 1 to 18 carbon atoms and a divalent alicyclic hydrocarbon group. Examples of the linear or branched alkylene group having 1 to 18 carbon atoms include a methylene group, a methylmethylene group, a dimethylmethylene group, an ethylene group, a propylene group and a trimethylene group. Examples of the above divalent alicyclic hydrocarbon group include a divalent cycloalkylene group (including a cycloalkylidene group) such as 1,2-cyclopentylene group, a 1,3-cyclopentylene group, a cyclopentylidene group, a 1,2-cyclohexylene group, a 1,3-cyclohexylene group, a 1,4-cyclohexylene group and a cyclohexylidene group.

Examples of the alkenylene group in the alkenylene group whose carbon-carbon double bonds are wholly or partially epoxidized (sometimes referred to as an "epoxidized alkenylene group") include a linear or branched alkenylene group having 2 to 8 carbon atoms such as a vinylene group, a propenylene group, a 1-butenylene group, a 2-butenylene group, a butadienylene group, a pentenylene group, a hexenylene group, a heptenylene group and an octenylene group. As the epoxidized alkenylene group, particularly, an alkenylene group whose carbon-carbon double bonds are wholly epoxidized is preferable, and an alkenylene group having 2 to 4 carbon atoms, whose carbon-carbon double bonds are

wholly epoxidized is more preferable.

As the linking group X, particularly, a linking group having an oxygen atom is preferable. Specific examples thereof include -CO-, -O-CO-O-, -COO-, -O-, - CONH-, an epoxidized alkenylene group; a group formed by linking a plurality of these groups; and a group formed by linking one or two or more of these groups and one or two or more divalent hydrocarbon groups (however, a group represented by the formula (6) is not included). Examples of the divalent hydrocarbon group include the same as defined above.

Typical examples of an alicyclic epoxy compound represented by the above formula (I) include compounds represented by the following formulas (I-1) to (I-10). Note that, in the following formulas (I-5) and (I-7), a and b each represent an integer of 1 to 30. In the following formula (I-5), R represents an alkylene group having 1 to 8 carbon atoms, more specifically, a linear or branched alkylene group such as a methylene group, an ethylene group, a propylene group, an isopropylene group, a butylene group, an isobutylene group, an s-butylene group, a pentylene group, a hexylene group, a heptylene group and an octylene group. Of them, linear or branched alkylene groups having 1 to 3 carbon atoms such as a methylene group, an ethylene group, a propylene group and an isopropylene group are preferable. In the following formulas (I-9) and (I-10), c1 to c6 each represent an integer of 1 to 30. Other than those mentioned above, for example, 1,2-bis(3,4-epoxycyclohexyl)ethane and 1,2-epoxy-1,2-bis(3,4-epoxycyclohexan-1-yl)ethane are mentioned.

[Formula 15]

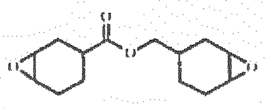
(I-1)

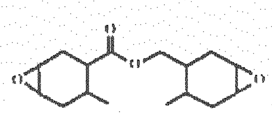
(I-2)

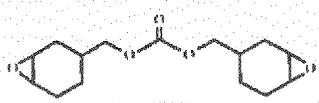
(I-3)

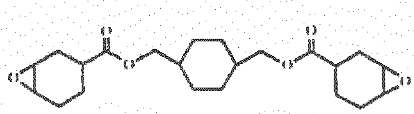
(I-4)

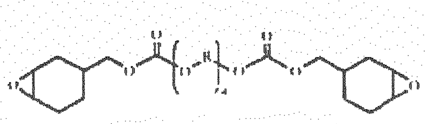
(I-5)

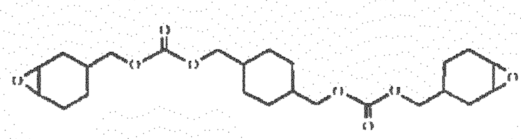
(I-6)

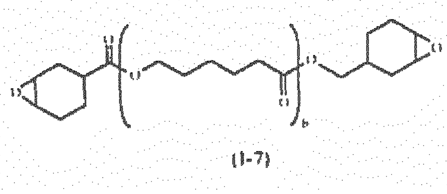
(I-7)

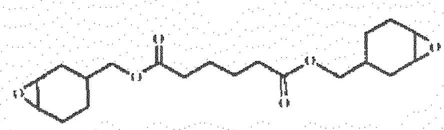
(I-8)

[Formula 16]

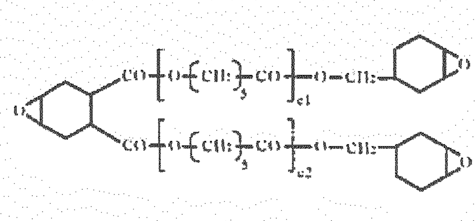
(I-9)

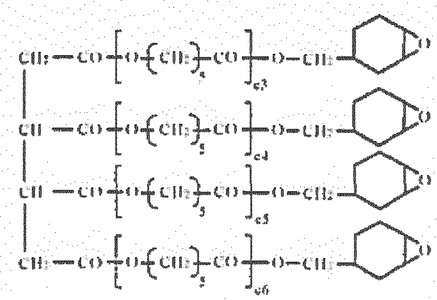
(I-10)

Examples of the compound (ii) in which an epoxy group is directly bound to an alicyclic ring via a single bond, include a compound represented by the following formula (II). [Formula 17]

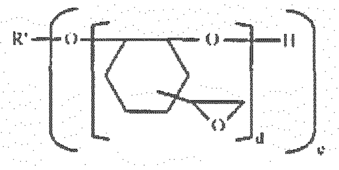

(II)

In the formula (II), R' represents a group obtained by removing e number of -OH from the structural formula of an e-valent alcohol; and d and e each represent a natural number. Examples of the e-valent alcohol represented by $[R'-(OH)_{e}]$ include a polyhydric alcohol such as 2,2-bis(hydroxymethyl)-1-butanol (alcohols having 1 to 15 carbon atoms). The reference symbol e preferably represents 1 to 6 and d preferably represents 1 to 30. If e is 2 or larger, the natural numbers represented by d within individual brackets (outer brackets) may be the same or different. Specific examples of the compound represented by the above formula (II) include 1,2-epoxy-4-(2-oxiranyl)cyclohexane adduct of 2,2-bis(hydroxymethyl)-1-butanol [e.g., "EHPE3150" (trade name, manufactured by Daicel Corporation)].

Examples of the compound (iii) having an alicyclic ring and a glycidyl group include a compound obtained by hydrogenating a bisphenol A epoxy compound (hydrogenated bisphenol A epoxy compound) such as 2,2-bis[4-(2,3-epoxypropoxy)cyclohexyl]propane and 2,2-bis[3,5-dimethyl-4-(2,3-epoxypropoxy)cyclohexyl]propane; a compound obtained by hydrogenating a bisphenol F epoxy compound (hydrogenated bisphenol F epoxy compound) such as bis[o,o-(2,3-epoxypropoxy)cyclohexyl]methane, bis[o,p-(2,3-epoxypropoxy)cyclohexyl]methane, bis[p,p-(2,3-epoxypropoxy)cyclohexyl]methane and bis[3,5-dimethyl-4-(2,3-epoxypropoxy)cyclohexyl]methane; a hydrogenated biphenol epoxy compound; a hydrogenated phenol Novolak epoxy compound; hydrogenated cresol Novolak epoxy compound; a hydrogenated cresol Novolak epoxy compound of bisphenol A; a hydrogenated naphthalene epoxy compound; and a hydrogenated aromatic glycidyl ether epoxy compound such as a hydrogenated epoxy compound obtained by hydrogenating an epoxy compound obtained from trisphenolmethane.

Examples of the aromatic glycidyl ether epoxy compound described above include a bisphenol A epoxy compound, a bisphenol F epoxy compound, a biphenol epoxy compound, a phenol Novolak epoxy compound, a cresol Novolak epoxy compound, a bisphenol A cresol Novolak epoxy compound, a naphthalene epoxy compound and an epoxy compound obtained from trisphenolmethane.

Examples of the aliphatic polyhydric alcohol polyglycidyl ether described above include a polyglycidyl ether of an aliphatic polyhydric alcohol such as glycerin, tetramethylene glycol, sorbitol, sorbitan, polyglycerin, pentaerythritol, tetramethylene glycol, hexamethylene glycol, trimethylolpropane, polyethylene glycol and polypropylene glycol.

Examples of the oxetane compound described above include 3,3-bis(vinyloxymethyl)oxetane, 3-ethyl-3-(2-ethylhexyloxymethyl)oxetane, 3-ethyl-3-(hydroxymethyl)oxetane, 3-ethyl-3-[(phenoxy)methyl]oxetane, 3-ethyl-3-(hexyloxymethyl)oxetane, 3-ethyl-3-(chloromethyl)oxetane, 3,3-bis(chloromethyl)oxetane, bis{[1-ethyl(3-oxetanyl)]methyl} ether, 4,4'-bis[(3-ethyl-3-oxetanyl)methoxymethyl]bicyclohexyl, 1,4-bis[(3-ethyl-3-oxetanyl)methoxymethyl]cyclohexane, 1,4-bis{[(3-ethyl-3-oxetanyl)methoxy]methyl}benzene and 3-ethyl-3-{[(3-ethyloxetan-3-yl)methoxy]methyl}oxetane.

Examples of the vinyl ether compound described above include 2-hydroxyethyl vinyl ether, 3-hydroxypropyl vinyl ether, 2-hydroxypropyl vinyl ether, 2-hydroxy-isopropyl vinyl ether, 4-hydroxybutyl vinyl ether, 3-hydroxybutyl vinyl ether, 2-hydroxybutyl vinyl ether, 3-hydroxyisobutyl vinyl ether, 2-hydroxyisobutyl vinyl ether, 1-methyl-3-hydroxypropyl vinyl ether, 1-methyl-2-hydroxypropyl vinyl ether, 1-hydroxymethylpropyl vinyl ether, 4-hydroxycyclohexyl vinyl ether, 1,6-hexanediol monovinyl ether, 1,6-hexanediol divinyl ether, 1,4-cyclohexanedimethanol monovinyl ether, 1,4-cyclohexane dimethanol divinyl ether, 1,3-cyclohexanedimethanol monovinyl ether, 1,3-cyclohexane dimethanol divinyl ether, 1,2-cyclohexanedimethanol monovinyl ether, 1,2-cyclohexane dimethanol divinyl ether, p-xylylene glycol monovinyl ether, p-xylylene glycol divinyl ether, m-xylylene glycol monovinyl ether, m-xylylene glycol divinyl ether, o-xylene glycol monovinyl ether, o-xylylene glycol divinyl ether, diethylene glycol monovinyl ether, diethylene glycol divinyl ether, triethylene glycol monovinyl ether, triethylene glycol divinyl ether, tetraethylene glycol monovinyl ether, tetraethylene glycol divinyl ether, penta-ethylene glycol monovinyl ether, penta-ethylene glycol divinyl ether, oligo-ethylene glycol monovinyl ether, oligo-ethylene glycol divinyl ether, polyethylene glycol monovinyl ether, polyethylene glycol divinyl ether, dipropylene glycol monovinyl ether, dipropylene glycol divinyl ether, tripropylene glycol monovinyl ether, tripropylene glycol divinyl ether, tetra-propylene glycol monovinyl ether, tetra-propylene glycol divinyl ether, penta-propylene glycol monovinyl ether, penta-propylene glycol divinyl ether, oligo-propylene glycol monovinyl ether, oligo-propylene glycol divinyl ether, polypropylene glycol monovinyl ether, polypropylene glycol divinyl ether, isosorbide divinyl ether, oxanorbornene divinyl ether, phenyl vinyl ether, n-butyl vinyl ether, octyl vinyl ether, cyclohexyl vinyl ether, hydroquinone divinyl ether and 1,4-butanediol divinyl ether.

In the curable epoxy resin composition of the present invention, as the other cationic curable compound, compounds can be used alone or in combination of two or more. As the other cationic curable compound, a commercially available compound can be used.

In the curable epoxy resin composition of the present invention, the content (blending quantity) of the other cationic curable compound, although it is not particularly limited, is preferably 90 wt% or less (e.g., 0 to 90 wt%) relative to the total amount (100 wt%) of cationic curable compounds, and more preferably 80 wt% or less.

[Additive]

The curable epoxy resin composition of the present invention may comprise various additives other than the above components as long as the effects of the present invention are not impaired. If, e.g., a compound having a hydroxyl group such as ethylene glycol, diethylene glycol, propylene glycol and glycerin is added as the additive, reaction is allowed to proceed moderately. Other than this, as long as the effects of the present invention are not impaired, conventional additives such as a curing aid, an organosiloxane compound, a metal oxide particle, a rubber particle, silicone or fluorine based defoamer, a silane coupling agent, a filler, a plasticizer, a leveling agent, an antistatic agent, a release agent, a flame retardant, a colorant, an antioxidant, an ultraviolet absorber, an ion adsorbent, a pigment and a dye can be used.

The curable epoxy resin composition of the present invention, although it is not particularly limited, is prepared by stirring and mixing the aforementioned individual components while heating, if necessary. Note that the curable epoxy resin composition of the present invention may be a single liquid composition in which individual components are previously mixed and can be directly used as it is, or may be a multiple liquid composition (e.g., two liquid composition) consisting of two or more separate portions (each portion may be a mixture of two or more components), which is used by blending the portions in a predetermined ratio just before use. As the stirring and mixing method, although it is not particularly limited, for example, known or conventional stirring and mixing means including a mixer such as a dissolver and a homogenizer, a kneader, a roll, a beads mill and a planetary centrifugal mixer can be used. After stirring and mixing, defoaming may be carried out under vacuum.

<Cured product>

A cured product having a high glass-transition temperature and particularly having excellent balance between heat resistance and transparency can be obtained by curing the curable epoxy resin composition of the present invention (the cured product obtained by curing the curable epoxy resin composition of the present invention will be sometimes referred to as "the cured product of the present invention"). Particularly in the case where the curable epoxy resin composition of the present invention comprises a curing catalyst (C) as an essential component, since the cure rate is higher, a cured product can be provided with high productivity. The temperature (curing temperature) in the case where curing is made by heating, although it is not particularly limited, is preferably 45 to 200°C, more preferably 50 to 190°C and further preferably 55 to 180°C. The time (curing time) for heating in curing, although it is not particularly limited, is preferably 30 to 600 minutes, more preferably 45 to 540 minutes and further preferably 60 to 480 minutes. If the curing temperature and curing time are lower than the lower limit value of the above range, curing becomes insufficient. Conversely, if the curing temperature and curing time are higher than the upper limit value of the above range, a resin component can be sometimes decomposed. Thus, both cases are not preferable. The curing conditions vary depending upon individual conditions, more specifically, can be appropriately controlled by reducing the curing time if the curing temperature is increased or by increasing the curing time if the curing temperature is lowered. The curable epoxy resin composition of the present invention (e.g., a photocationic polymerization initiator is comprised as the curing catalyst (C)) can be cured by irradiation of an active energy ray. Examples of the active energy ray to be used include infrared ray, visible light, ultraviolet light, X-ray, electron beam, $\alpha$ ray, $\beta$ ray and $\gamma$ ray. Of them, ultraviolet light is preferable since it is excellent in handling. Conditions for irradiating the curable epoxy resin composition of the present invention with an active energy ray vary depending upon e.g., the type of active energy ray and shape of a cured product. Although the conditions are not particularly limited, for example, if ultraviolet light is applied, the irradiation intensity thereof is preferably set to be, for example, about 0.1 to 1000 $mW/cm^2$ (more preferably 1 to 500 $mW/cm^2$) and the irradiation time is set to be, for example, about 1 to 120 seconds and preferably 3 to 60 seconds. Note that curing can be made in a single step or in multiple steps of two or more steps. Furthermore, in curing, heating and irradiation with an active energy ray can be used in combination.

The curable epoxy resin composition of the present invention can be applied to various uses including a coating agent, a hard coating agent, an ink, an adhesive, a sealant, a sealing agent, a resist, a composite material [for example, fiber reinforced plastic (FRP) such as CFRP and GFRP], a transparent substrate, a transparent film or sheet, an optical material (e.g., an optical lens), a stereolithographic material and an electronic material (e.g., an electronic paper, a touch panel, a solar cell substrate, an optical waveguide, a light guide plate, a holographic memory), a machine-part material,

an electrical-part material, an auto-part material, a civil engineering and construction material, a molding material, a plastic forming material and a solvent (e.g., reactive diluents).

## Examples

The present invention will be more specifically described based on Examples; however the present invention is not limited by these Examples. Note that in the curable epoxy resin compositions shown in Tables 1 to 3, the mixing ratios of individual components are expressed by parts by weight. In Tables 1 to 3, the symbol "-" means that the component was not blended. Furthermore, blending quantities of "SunaidSI-100L" (trade name) in Table 2 and "CPI-100P" (trade name) in Table 3 each were expressed by the amount of the product, itself.

### Production Example 1

[Production of tetrahydrobenzyl methane sulfonate]

To a 5 L reactor, tetrahydrobenzyl alcohol (400 g, 3.57 mol) represented by the following formula (3-1), triethylamine (379 g, 3.74 mol) and toluene (927 mL) were added. After the atmosphere of the reactor was replaced with nitrogen, the reaction mixture was cooled to 5°C.

To the reaction mixture, a toluene (461 mL) solution of methanesulfonyl chloride (429 g, 3.74 mol) represented by the following formula (5-1) was added dropwise at a temperature in the range of 5 to 10°C and the reaction mixture was allowed to standstill for 30 minutes. Thereafter, ion-exchanged water was added to the reaction system to terminate the reaction. An organic layer was extracted and washed once with an aqueous sodium hydrogen carbonate solution, once with a 1 M aqueous hydrochloric acid solution and once with ion-exchanged water. The resulting organic layer was concentrated to obtain tetrahydrobenzyl methane sulfonate (yield: 98%) represented by the following formula (4-1).

[Formula 18]

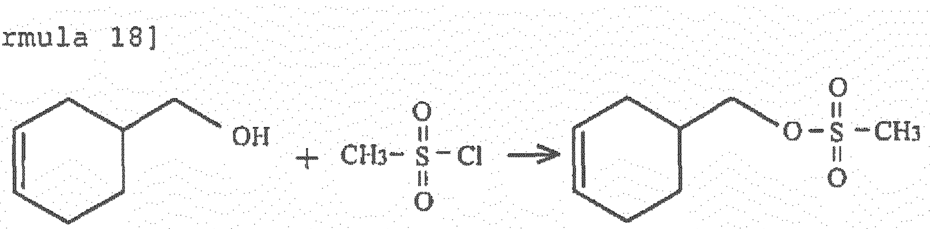

(3-1) (5-1) (4-1)

### Example 1

[Production of ditetrahydrobenzyl ether]

To a 5 L reactor, sodium hydroxide (granule) (499 g, 12.48 mol) and toluene (727 mL) were added. After the atmosphere of the reactor was replaced with nitrogen, a toluene (484 mL) solution of tetrahydrobenzyl alcohol (420 g, 3.74 mol) represented by the following formula (3-1) was added. The reaction mixture was allowed to stand still at 70°C for 1.5 hours. Subsequently, tetrahydrobenzyl methane sulfonate (419 g, 2.20 mol) obtained in Production Example 1 and represented by the following formula (4-1) was added. After the reaction mixture was allowed to stand still for 3 hours under reflux, the mixture was cooled to room temperature. Water (1248 g) was added to the mixture to terminate the reaction and the mixture was allowed to separate into layers. The organic layer separated was concentrated and then subjected to distillation under reduced pressure to obtain ditetrahydrobenzyl ether represented by the following formula (2-1) as a colorless transparent liquid (conversion rate: 99%, selectivity: 98%, yield: 85%). The $^1$H-NMR spectrum of the ditetrahydrobenzyl ether obtained was measured.

$^1$H-NMR($CDCl_3$):$\delta$1.23-1.33(m,2H),1.68-1.94(m,6H),2.02-2.15(m, 6H),3.26-3.39(m,9H),5.63-7.70(m,4H)

[Formula 19]

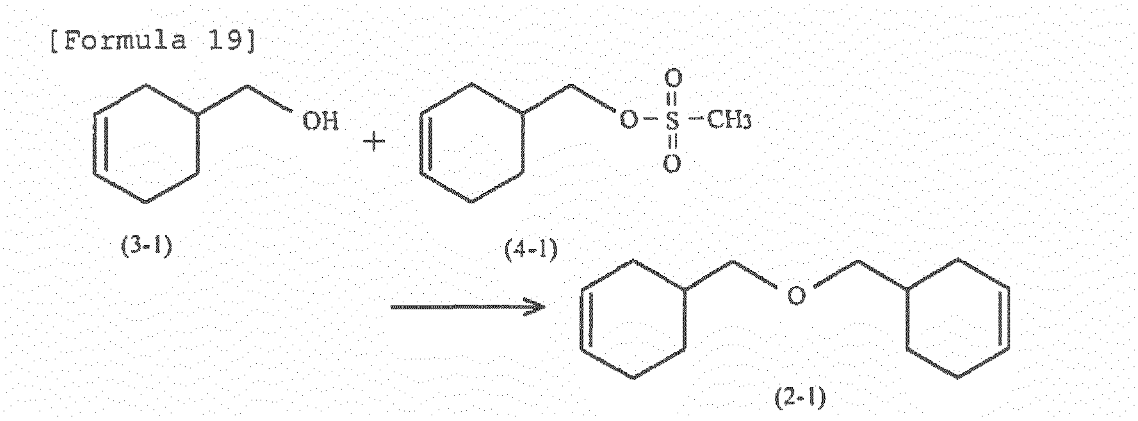

Example 2

[Production of ditetrahydrobenzyl ether]

To a 50 mL reactor, sodium hydroxide (granule) (1.78 g, 0.027 mol) and cyclohexane (3.85 mL) were added. After the atmosphere of the reactor was replaced with nitrogen, a cyclohexane (2.89 mL) solution of tetrahydrobenzyl alcohol (1.5 g, 0.013 mol) was added to the reaction mixture and the mixture was allowed to stand still at 70°C for 1.5 hours.
To the reaction mixture, tetrahydrobenzyl methane sulfonate (5.1 g, 0.027 mol) obtained in Production Example 1 was added. The reaction mixture was allowed to stand still for 3 hours under reflux and cooled to room temperature. Water (4.46 g) was then added to the mixture to terminate the reaction and the mixture was allowed to separate into layers.
The organic layer separated was analyzed by gas chromatography, and it was confirmed that ditetrahydrobenzyl ether was obtained with a conversion rate of 98% and a selectivity of 98%.

Example 3

[Production of ditetrahydrobenzyl ether]

The reaction was carried out in the same scale and manner as in Example 2 except that tetrahydrobenzyl bromide was used in place of tetrahydrobenzyl methane sulfonate and the reaction was allowed to stand still for 17 hours in place of 3 hours. As a result, ditetrahydrobenzyl ether was obtained with a conversion rate of 80% and a selectivity of 98%.

Example 4

[Production of ditetrahydrobenzyl ether]

The reaction was carried out in the same scale and manner as in Example 2 except that tetrahydrobenzyl chloride was used in place of tetrahydrobenzyl methane sulfonate and the reaction was allowed to stand still for 5 hours in place of 3 hours. As a result, ditetrahydrobenzyl ether was obtained with a conversion rate of 5%, and a selectivity of 98%.

Example 5

[Production of bis(3,4-epoxycyclohexylmethyl)ether]

Ditetrahydrobenzyl ether (200 g, 0.97 mol) obtained in Example 1 and represented by the following formula (2-1), 20% SP-D (acetic acid solution) (0.39 g) and ethyl acetate (669 mL) were added to a reactor. The temperature of the reaction mixture was raised to 40°C. Subsequently, 29.1% peracetic acid (608 g, corresponding to 2.4 mol relative to ditetrahydrobenzyl ether (1 mol)) was added dropwise over 5 hours and the reaction mixture was allowed to stand still for 3 hours. Thereafter, the resultant organic layer was washed three times with an aqueous alkaline solution and twice with ion-exchanged water, and then subjected to distillation under reduced pressure to obtain bis(3,4-epoxycyclohexylmethyl)ether represented by the following formula (1-1) as a colorless transparent liquid (yield: 77%).
[Formula 20]

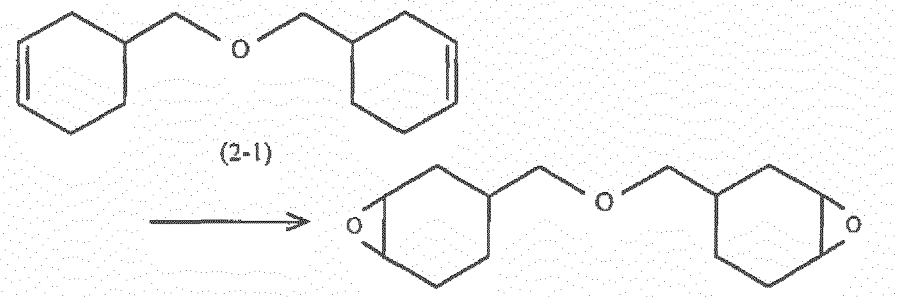

(2-1)

(1-1)

Example 6

[Production of curable epoxy resin composition and cured product thereof]

A curing agent "RIKACID MH-700F" (trade name, manufactured by New Japan Chemical Co., Ltd.), a curing accelerator, "U-CAT 12XD" (trade name, manufactured by San-Apro Ltd.) and a diluent, ethylene glycol (manufactured by Wako Pure Chemical Industries Ltd.) were blended in accordance with the mixing ratio (unit: parts by weight) shown in Table 1, homogeneously mixed and defoamed by use of a planetary centrifugal mixer (trade name "Awatori Neritaro AR-250", manufactured by THINKY) to obtain a curing agent composition.

Subsequently, bis(3,4-epoxycyclohexylmethyl)ether obtained in Example 5 and the curing agent composition obtained above were blended in accordance with the mixing ratio (unit: parts by weight) shown in Table 1, homogeneously mixed and defoamed by use of the planetary centrifugal mixer (trade name '"Awatori Neritaro AR-250", manufactured by THINKY) to produce a curable epoxy resin composition.

The curable epoxy resin composition obtained above was put in shaping dies (mold forms having a thickness of 4 mm, 3 mm and 0.5 mm), placed in an oven for curing resin and heated in the curing conditions [100°C for 2 hours, subsequently 150°C for 3 hours] shown in Table 1. In this manner, the resin composition was cured to obtain a cured product.

Comparative Examples 1 to 3

[Production of curable epoxy resin composition and cured product thereof]

Curable epoxy resin compositions and cured products thereof were produced in the same manner as in Example 6 except that the type and amount of epoxy compound, the composition of curing agent composition and curing conditions were changed to those shown in Table 1.

Example 7

[Production of curable epoxy resin composition and cured product thereof]

Bis(3,4-epoxycyclohexylmethyl)ether obtained in Example 5 and a curing catalyst "SunaidSI-100L" (trade name, manufactured by Sanshin Chemical Industry Co., Ltd.) were blended in accordance with the mixing ratio (unit: parts by weight) shown in Table 2, homogeneously mixed and defoamed by use of a planetary centrifugal mixer (trade name "Awatori Neritaro AR-250", manufactured by THINKY) to obtain a curable epoxy resin composition.

The curable epoxy resin composition obtained above was put in shaping dies (mold forms having a thickness of 4 mm, 3 mm and 0.5 mm), placed in an oven for curing resin and heated in the curing conditions [55°C for 2 hours, subsequently 150°C for 2 hours] shown in Table 2. In this manner, the resin composition was cured to obtain a cured product.

Comparative Examples 4 to 6

[Production of curable epoxy resin composition and cured product thereof]

Curable epoxy resin compositions and cured products thereof were produced in the same manner as in Example 7 except that the type and amount of epoxy compound and curing conditions were changed to those shown in Table 2.

<Evaluation>

The curable epoxy resin compositions and cured products obtained in Examples 6, 7 and Comparative Examples 1 to 6 were subjected to the following evaluation tests.

[Viscosity of curable epoxy resin composition (25°C)]

The viscosity of each of the curable epoxy resin compositions obtained in Examples 6, 7 and Comparative Examples 1 to 6 at 25°C was measured by a digital viscometer (model number "DVU-EII", manufactured by Tokimec, Inc.) in the conditions of rotor: standard 1°34' × R24, temperature: 25°C, rotation speed: 0.5 to 10 rpm.

[Pot life]

The curable epoxy resin compositions obtained in Examples 6, 7 and Comparative Examples 1 to 6 were heated at 50°C for predetermined time periods (2 hours, 3 hours, or 4 hours) shown in Tables 1 and 2 and the viscosity of them at 25°C after heating was measured by a digital viscometer (model number "DVU-EII" manufactured by Tokimec, Inc.) in the conditions of rotor: standard 1°34' × R24, temperature: 25°C, rotation speed: 0.5 to 10 rpm.

Note that when viscosity values before and after heating are compared, if the degree of increase in viscosity by heating is smaller, the pot life is meant be longer.

[Gelation time]

The curable epoxy resin compositions obtained in Examples 6, 7 and Comparative Examples 1 to 6 were measured by a gel time tester (manufactured by Yasuda Seiki Seisakusho Ltd.) in the conditions of rotor: diameter Φ5 × 110 mm, test tube: outer diameter Φ12 × 90 mm, oil: SRX 310 (heated to predetermined temperature (150°C or 120°C) shown in Tables 1 and 2). The time required for gelatinizing a sample (time at which a magnet immobilizing a rotor was removed due to a viscosity increase) was specified as gelation time.

[Heat resistance (TMA)]

The glass-transition temperature (Tg (TMA)) of each of the cured products obtained in Examples 6, 7 and Comparative Examples 1 to 6 was obtained by using a TMA measuring device ("TMA/SS100", manufactured by SII NanoTechnology Inc.) by the method in accordance with JIS K7197 as follows. The coefficient of thermal expansion was measured under a nitrogen atmosphere, at a temperature increase rate of 5°C/minute, in the measurement temperature range of 30 to 250°C. Thereafter, tangent lines were drawn at points of a curve before and after the glass-transition point. The intersection of these tangent lines is defined as the glass-transition temperature. The linear expansion coefficient of each of the cured products obtained in Examples and Comparative Examples was obtained by specifying the gradient of the linear line on the low-temperature side of the glass-transition temperature obtained above as $\alpha 1$, and the gradient of the linear line on the high-temperature side of the glass-transition temperature as $\alpha 2$.

[Heat resistance (DMA)]

From each of the cured products (thickness: 0.5 mm) obtained in Example 6 and Comparative Examples 1 to 3, test pieces having a size of a thickness of 0.5 mm × a width of 8 mm × a length of 40 mm were excised out. The peak top temperature (Tg (DMA-tan5)) of a loss tangent (tan5) of the test piece and the glass-transition onset temperature (Tg (DMA-E')) of a storage elastic modulus (E') were measured by using a dynamic viscoelasticity measuring device (DMA) (manufactured by Seiko Instruments Inc.). Note that measurement was carried out in the conditions: under a nitrogen stream, measurement temperature range: -50 to 300°C, temperature increase rate: 3°C/minute and deformation mode: tensile mode.

[Mechanical characteristics (bending test)]

The cured products (obtained in Example 6 and Comparative Examples 1 to 3) having a thickness of 4 mm × a width of 10 mm × a length of 80 mm were used as samples and subjected to a three-point bending test performed by use of a Tensilon universal testing machine (manufactured by Orientec Co., Ltd.) in the conditions of edge span: 67 mm, bending rate: 2 mm/minute. In this manner, bending strength, bending modulus and bending elongation of the cured products were measured.

[Transparency]

The light transmittance of each of the cured products obtained in Examples 6, 7 and Comparative Examples 1 to 6 (thickness: 3 mm) at a wavelength of 400 nm was measured by use of a spectrophotometer (trade name, "UV-2450", manufactured by Shimadzu Corporation) and expressed as "transmittance (400 nm) [150°C × 0h]".

Subsequently, the cured product was heated at 150°C. The light transmittance 24 hours after initiation of heating at a wavelength of 400 nm (expressed as "transmittance (400 nm) [150°C × 24h]") and the light transmittance 50 hours after initiation of heating at a wavelength of 400 nm (expressed as "transmittance (400 nm) [150°C × 50h]") were measured in the same manner as above.

[Water absorption]

Each of the cured products (thickness: 3 mm) obtained in Example 6 and Comparative Examples 1 to 3 was dried in the conditions: 50°C and 24 hours and then cooled in a desiccator (containing silica gel), and the weight of the blank (M1) was measured. Thereafter, the cured product was allowed to stand still in water in the conditions: 23°C and 24 hours. After taken out, the cured product was wiped with gauze and the weight thereof was measured within one minute. This was regarded as the weight (M2) of the cured product after water absorption. The water absorption of the cured product was measured in accordance with the following expression.

$$\text{Water absorption (\%)} = \{(M2-M1)/M1\} \times 100$$

[Table 1]

(Table 1)

| | | | | Example 6 | Comparative Example 1 | Comparative Example 2 | Comparative Example 3 |
|---|---|---|---|---|---|---|---|
| Curable epoxy resin composition | Epoxy compound | Bis(3,4-epoxycyclohexylmethyl)ether | parts by weight | 100 | - | - | - |
| | | Celloxide 2021P | parts by weight | - | 100 | 50 | - |
| | | EHPE3150 | parts by weight | - | - | 50 | - |
| | | YD-128 | parts by weight | - | - | - | 100 |
| | Curing agent | RIKACID MH-700F | parts by weight | 119.6 | 112.8 | 97.2 | 79.3 |
| | Diluent | EG | parts by weight, | 2.0 | 2.0 | 2.0 | 2.0 |
| | Curing accelerator | U-CAT 12XD | parts by weight | 0.5 | 0.5 | 0.5 | 0.5 |
| Curing conditions | | | | 100°C*2h 150°C×3h | 100°C×2h 150°C×2h | 100°C×2h 150°C×2h | 100°C×2h 150°C×2h |
| Evaluation results | Viscosity | Viscosity 125°C) | mPa·s | 140 | 213 | 2613 | 2528 |
| | Pot life | Viscosity (25°C) (50°C×4h) | mPa·s | 290 | 319 | 25140 | 5606 |
| | Gelation time | Gelation time (150°C) | min | 7.7 | 7.7 | 5.2 | 6.4 |
| | Heat resistance (TMA) (5°C/min) | Tg (TMA) | °C | 190.7 | 188.5 | 185.6 | 126.9 |
| | | α1 | ppm/°C | 74.8 | 74.4 | 85.2 | 71.1 |
| | | α2 | ppm/°C | 167.2 | 172.4 | 167.4 | 178.9 |
| | Heat resistance (DMA) (3°C/min) | Tg (DMA-tanδ) | °C | 202.4 | 177.5 | 215.1 | 114.1 |
| | | Tg (DMA-E') | °C | 177.5 | 152.8 | 161.4 | 93.5 |
| | Mechanical characteristic (bending test) | Bending strength | MPa | 128.2 | 128.8 | 96.3 | 124.1 |
| | | Bending modulus | MPa | 2877.1 | 2975.5 | 3173.8 | 2673.5 |
| | | Bending elongation | % GL | 5.3 | 5.1 | 3.1 | 6.7 |

(continued)

| | | | | Example 6 | Comparative Example 1 | Comparative Example 2 | Comparative Example 3 |
|---|---|---|---|---|---|---|---|
| | Transparency | Transmittance (400nm) [150°C×0h] | % | 90.9 | 89.0 | 88.1 | 78.5 |
| | | Transmittance (400nm) [150°C×24h] | % | 84.2 | 83.4 | 80.6 | 47.0 |
| | | Transmittance (400nm) (150°C-50h) | % | 77.8 | 77.6 | 75.9 | 41.7 |
| | Water absorption | Transmittance [23°C×24h] | % | 0.32 | 0.37 | 0.40 | 0.18 |

[Table 2]

| | | | | Example 7 | Comparative Example 4 | Comparative Example 5 | Comparative Example 6 |
|---|---|---|---|---|---|---|---|
| Curable epoxy resin composition | Epoxy compound | Bis(3,4-epoxycyclohexylmethyliether | parts by weight | 100 | - | - | - |
| | | Celloxide 2021P | parts by weight | - | 100 | 50 | - |
| | | EHPE3150 | parts by weight | - | - | 50 | - |
| | | YD-128 | parts by weight | - | - | - | 100 |
| | Curing catalyst | Sunaid SI-100L | parts by weight | 0.60 | 0.60 | 0.60 | 0.60 |
| Curing conditions | | | | 55°C×2h 150°C×2h | 65°C×2h 150°C×2h | 65°C×2h 150°C×2h | 120°C×1h 180°C×1h |
| Evaluation results | Viscosity | Viscosity (25°C) | mPa·s | 107 | 238 | 46007 | 13756 |
| | Pot life | Viscosity (25°C) [50°C×2h) | mPa·s | 108 | 238 | 42409 | 13468 |
| | | Viscosity 125°C) [150°C×3h] | mPa·s | 115 | 235 | - | - |
| | Gelation time | Gelation time [120°C] | min | 0.6 | 1.4 | 1.6 | 10.5 |
| | Heat resistance (TMA) (5°C/min) | Tg(TMA) | °C | 163.5 | 162.8 | 165.5 | 155.1 |
| | | $\alpha 1$ | ppm/°C | 70.7 | 84.6 | 74.5 | 74.2 |
| | | $\alpha 2$ | ppm/°C | 121.9 | 137.6 | 129.3 | 160.3 |
| | Transparency | Transmittance [400nm] [150°C×0h] | % | 83.3 | 82.7 | 60.6 | 21.7 |
| | | Transmittance (400nm)[150°C×24h] | % | 65.3 | 58.3 | 42.2 | 0.0 |
| | | Transmittance (400nm) [150°C×50h] | % | 49.1 | 42.5 | 28.7 | 0.0 |

Note that the brevity codes in Tables 1 and 2 represent the followings.

(Epoxy compound)

Celloxide 2021P: trade name, "Celloxide 2021P" [3,4-epoxycyclohexylmethyl(3,4-epoxy)cyclohexane carboxylate, manufactured by Daicel Corporation]
EHPE 3150: trade name, "EHPE3150", [1,2-epoxy-4-(2-oxyranyl)cyclohexene adduct of 2,2-bis(hydroxymethyl)-1-butanol (Mw: about 2000), manufactured by Daicel Corporation]
YD-128: trade name, "YD-128" [bisphenol A epoxy resin, manufactured by Nippon Steel Chemical Co., Ltd.]

(Curing agent)

RIKACID MH-700F: trade name, "RIKACID MH-700F" [4-methylhexahydrophthalic anhydride/hexahydrophthalic anhydride = 70/30, manufactured by New Japan Chemical Co., Ltd.]

(Curing accelerator)

U-CAT 12XD: trade name, "U-CAT 12XD" [manufactured by San-Apro Ltd.]

(Diluent)

EG: trade name, "ethylene glycol" [manufactured by Wako Pure Chemical Industries Ltd.]

(Curing catalyst)

Sunaid SI-100L: trade name "Sunaid SI-100L" [manufactured by Sanshin Chemical Industry Co., Ltd.]

As shown in Table 1, a cured product (Example 6) of the curable epoxy resin composition of the present invention containing the curing agent (B) as an essential component had high glass-transition temperature and high transparency, a low degree of reduction in transparency during heating and excellent balance between heat resistance and transparency. More specifically, the curable epoxy resin composition of the present invention (Example 6) had low viscosity and was excellent in handling compared to a composition containing no alicyclic epoxy compound (A), for example, compositions obtained in Comparative Examples 1 to 3; at the same time, the cured product of the invention had a high glass-transition temperature, excellent transparency and transparency maintaining rate during heating, compared to the cured products obtained in Comparative Examples 1 to 3.
In contrast, as shown in Table 2, the curable epoxy resin composition of the present invention (Example 7) containing the curing catalyst (C) as an essential component had a short gelation time and a high cure rate. The cured product (Example 7) obtained by curing the above curable epoxy resin composition had a high glass-transition temperature, high transparency, a low degree of reduction in transparency during heating and excellent balance between heat resistance and transparency. More specifically, the curable epoxy resin composition of the present invention (Example 7) had low viscosity and was excellent in handling, compared to a composition containing no alicyclic epoxy compound (A), for example, the compositions obtained in Comparative Examples 4 to 6. The cured product of the invention had a high glass-transition temperature, compared to the cured products obtained in Comparative Examples 4 and 6, and had excellent transparency and transparency maintaining rate during heating, compared to the cured products obtained in Comparative Examples 4 to 6.

Example 8

[Production of curable epoxy resin composition]

The bis(3,4-epoxycyclohexylmethyl)ether obtained in Example 5 and a photocationic polymerization initiator, "CPI-100P" (trade name, manufactured by San-Apro Ltd.) were blended in accordance with the mixing ratio (unit: parts by weight) shown in Table 3, homogeneously mixed and defoamed by use of a planetary centrifugal mixer (trade name "Awatori Neritaro AR-250", manufactured by THINKY) to produce a curable epoxy resin composition.

Comparative Example 7

[Production of curable epoxy resin composition]

A curable epoxy resin composition was produced in the same manner as in Example 8 except that the epoxy compound was changed to Celloxide 2021P, as shown in Table 3.

<Evaluation>

The curable epoxy resin compositions obtained in Example 8 and Comparative Example 7 were subjected to the following evaluation test.

[Measurement of photochemical reaction heat (light irradiation DSC)]

The photochemical reaction heat of each of the curable epoxy resin compositions obtained in Example 8 and Comparative Example 7 was measured by applying ultraviolet light of 360 nm at an irradiation intensity of 30 mW/cm$^2$ for 80 seconds by use of DSC ("DSC 6220" manufactured by SII NanoTechnology Inc.) and an ultraviolet irradiation apparatus ("UV-1 ultraviolet irradiation apparatus" manufactured by SEICO Electronics Industrial Co., Ltd.). The results are shown in Figure 1 and Table 3. Note that the DSC curve shown by a solid line in Figure 1 represents the curable epoxy resin composition obtained in Example 8; whereas the DSC curve shown by a dashed line represents the curable epoxy resin composition obtained in Comparative Example 7. Note that in Figure 1, the time point of 0 is the time at which irradiation of ultraviolet light was initiated.
[Table 3]

(Table 3)

| | | | | Example 8 | Comparative Example 7 |
|---|---|---|---|---|---|
| Curable epoxy resin composition | Epoxy compound | Bis(3,4-epoxycyclohexylmethyl)ether | parts by weight | 100 | - |
| | | Celloxide 2021P | parts by weight | - | 100 |
| | Photocationic polymerization initiator | CPI-100P | parts by weight | 5 | 5 |
| Evaluation results | Light irradiation DSC | Total calorific value | J/g | 423 | 221 |
| | | Calorific value per epoxy equivalent | J/eq | 50.4 | 28.7 |

Note that the brevity codes in Table 3 represent the followings.

(Epoxy compound)

Celloxide 2021P: trade name, "Celloxide 2021P" [3,4-epoxycyclohexylmethyl(3,4-epoxy)cyclohexane carboxylate, manufactured by Daicel Corporation]

(Curing catalyst)

CPI-100P: trade name, "CPI-100P" [photocationic polymerization initiator, manufactured by San-Apro Ltd.]
As is apparent from calorific values per epoxy equivalent shown in Figure 1 and Table 3, it was confirmed that the curable epoxy resin composition of the present invention (Example 8) has high reactivity to light and is excellent in curing properties at the irradiation amount of ultraviolet light (curing properties when irradiated with the same amount of ultraviolet light).

Industrial Applicability

The diepoxy compound (alicyclic epoxy compound (A)) of the present invention and the curable epoxy resin composition of the present invention comprising the diepoxy compound can be applied to various uses including a coating agent, a hard coating agent, an ink, an adhesive, a sealant, a sealing agent, a resist, a composite material [for example, fiber reinforced plastic (FRP) such as CFRP and GFRP], a transparent substrate, a transparent film or sheet, an optical material (e.g., an optical lens), a stereolithographic material and an electronic material (e.g., an electronic paper, a touch panel, a solar cell substrate, an optical waveguide, a light guide plate, a holographic memory), a machine-part material, an electrical-part material, an auto-part material, a civil engineering and construction material, a molding material, a plastic forming material and a solvent (e.g., reactive diluents).

## Claims

1. A curable epoxy resin composition comprising an alicyclic epoxy compound (A) represented by the following formula (1):

[Formula 1]

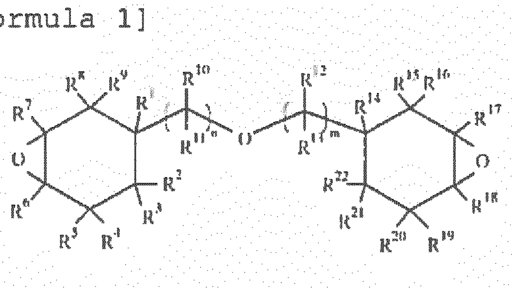

(1)

wherein $R^1$ to $R^{22}$, which may be the same or different, each represent a hydrogen atom, a methyl group or an ethyl group; and m and n, which may be the same or different, each represent an integer of 1 to 4, and a curing agent (B).

2. A curable epoxy resin composition comprising an alicyclic epoxy compound (A) represented by the following formula (1):

[Formula 2]

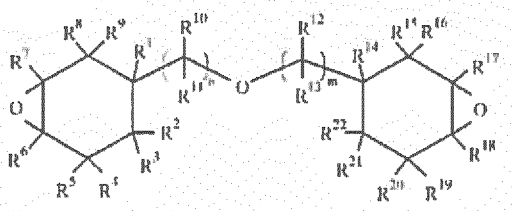

(1)

wherein $R^1$ to $R^{22}$, which may be the same or different, each represent a hydrogen atom, a methyl group or an ethyl group; and m and n, which may be the same or different, each represent an integer of 1 to 4,
and a curing catalyst (C).

3. The curable epoxy resin composition according to Claim 1 or 2, further comprising a curing accelerator (D).

4. A cured product obtained by curing the curable epoxy resin composition according to any one of Claims 1 to 3.

5. A diolefin compound represented by the following

[Formula 3]

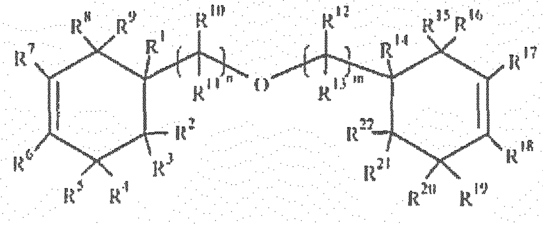

(2)

wherein $R^1$ to $R^{22}$, which may be the same or different, each represent a hydrogen atom, a methyl group or an ethyl group; and m and n, which may be the same or different, each represent an integer of 1 to 4.

**6.** A method for producing a diolefin compound represented by the following formula (2):

[Formula 6]

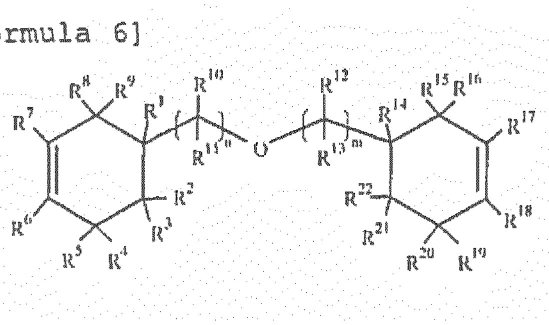

(2)

wherein $R^1$ to $R^{22}$, m and n are the same as defined above, by reacting a compound represented by the following formula (3):

[Formula 4]

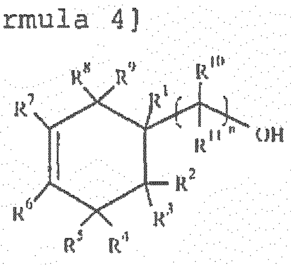

(3)

wherein $R^1$ to $R^{11}$, which may be the same or different, each represent a hydrogen atom, a methyl group or an ethyl group; and n represents an integer of 1 to 4,
and a compound represented by the following formula (4):

[Formula 5]

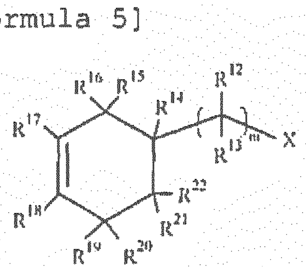

(4)

wherein $R^{12}$ to $R^{22}$, which may be the same or different, each represent a hydrogen atom, a methyl group or an ethyl group; X represents a halogen atom, a
benzenesulfonyloxy group, a p-toluenesulfonyloxy group, a methanesulfonyloxy group or a trifluoromethanesulfonyloxy group; and m represents an integer of 1 to 4.

7. The method for producing a diolefin compound according to Claim 6, wherein the reaction is carried out in the presence of a basic compound.

8. A method for producing a diepoxy compound represented by the following formula (1):

[Formula 8]

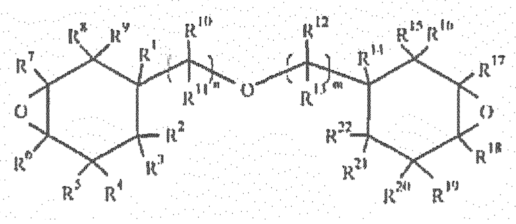

(1)

wherein $R^1$ to $R^{22}$, m and n are the same as defined above, by reacting a diolefin compound represented by the following formula (2):

[Formula 7]

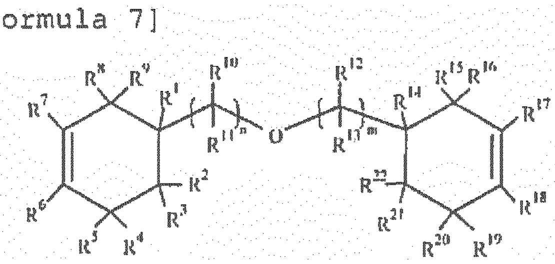

(2)

wherein $R^1$ to $R^{22}$, which may be the same or different, each represent a hydrogen atom, a methyl group or an ethyl group; and m and n, which may be the same or different, each represent an integer of 1 to 4,
and a peracid.

[Figure 1]

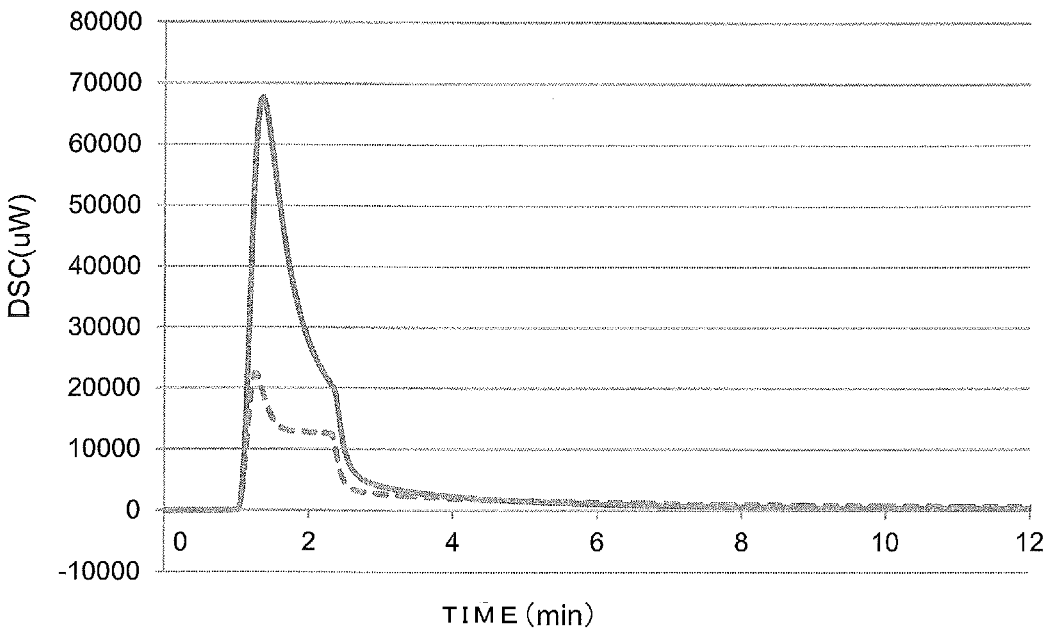
80000
70000
60000
50000
40000
30000
20000
10000
0
-10000
DSC(uW)
0
2
4
6
8
10
12
TIME (min)

**INTERNATIONAL SEARCH REPORT**

International application No.
PCT/JP2014/062217

A. CLASSIFICATION OF SUBJECT MATTER
*C08G59/24*(2006.01)i, *C07C41/16*(2006.01)i, *C07C43/162*(2006.01)i, *C07D303/28*(2006.01)i, *C08G59/68*(2006.01)i

According to International Patent Classification (IPC) or to both national classification and IPC

B. FIELDS SEARCHED

Minimum documentation searched (classification system followed by classification symbols)
C08G59/24, C07C41/16, C07C43/162, C07D303/28, C08G59/68

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)
CA(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y | US 3242108 A (Union Carbide Corp.), 22 March 1966 (22.03.1966), claims; column 3, lines 9 to 20; examples (Family: none) | 1,2,4-8<br>2-4,6,7 |
| X<br>Y | US 3198851 A (Union Carbide Corp.), 03 August 1965 (03.08.1965), claims; column 4, line 67 to column 5, line 45; examples (Family: none) | 2,4-8<br>1-4,6,7 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 June, 2014 (02.06.14) | 10 June, 2014 (10.06.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No. PCT/JP2014/062217

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | GB 989375 A (Union Carbide Corp.), 14 April 1965 (14.04.1965), claims; page 3, line 20 to page 5, line 6; examples<br>& US 3321550 A & GB 1037974 A<br>& FR 1305982 A & CH 405709 A<br>& NL 24222 C & NL 266665 A<br>& AT 243516 B | 2,4-8<br>1-4,6,7 |
| X<br>Y | JP 2007-023205 A (Konica Minolta Medical & Graphic, Inc.), 01 February 2007 (01.02.2007), claims; paragraphs [0025] to [0040]; examples (Family: none) | 2,4,5,8<br>1-4,6,7 |
| X<br>Y | WO 2007/029448 A1 (Konica Minolta Medical & Graphic, Inc.), 15 March 2007 (15.03.2007), claims; paragraph [0034]; examples<br>& US 2009/0137695 A1 & EP 1932891 A1 | 2,4,5,8<br>1-4,6,7 |
| X<br>Y<br>A | CN 102643254 A (CAS Guangzhou Chemistry Co., Ltd.), 22 August 2012 (22.08.2012), claims; examples (Family: none) | 5,8<br>1-4<br>6,7 |
| Y<br>A | JP 63-264625 A (Daicel Chemical Industries, Ltd.), 01 November 1988 (01.11.1988), claims; page 3, lower right column, lines 3 to 17 (Family: none) | 1,3,4<br>2,5-8 |
| A | Helmut Schweikl et al., Mutagenic activity of structurally related oxiranes and siloranes in Salmonella typhimurium, Mutation Research, 2002, 521, 19-27 | 1-8 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2013099967 A
- JP 2013100071 A
- JP 63264625 A
- JP 63012623 A
- JP 59011317 A